# EUROPÄISCHE PATENTSCHRIFT

(11) **EP 3 672 484 B1**
(45) Veröffentlichungstag und Bekanntmachung des Hinweises auf die Patenterteilung: **21.04.2021**
(21) Anmeldenummer: 19729228.7
(22) Anmeldetag: 03.06.2019
(51) Int. Cl.: A61B 5/11, A61B 5/00

(54) **VORRICHTUNG ZUM ERFASSEN, AUSWERTEN UND DIFFERENZIEREN VON TREMOR UND BRADYKINESE UND VERFAHREN ZUM ERFASSEN, AUSWERTEN UND DIFFERENZIEREN VON TREMOR**
APPARATUS FOR DETECTION, EVALUATION AND DIFFERENTIATION OF TREMOR AND BRADYKINESIA AND METHOD FOR DETECTION, EVALUATION AND DIFFERENTIATION OF TREMOR
APPAREIL POUR LA DÉTECTION, L'ÉVALUATION ET LA DIFFÉRENCIATION D'UN TREMBLEMENT ET D'UNE BRADYKINÉSIE ET PROCÉDÉ POUR LA DÉTECTION, L'ÉVALUATION ET LA DIFFÉRENCIATION D'UN TREMBLEMENT

(43) Veröffentlichungstag der Anmeldung: 01.07.2020
(73) Patentinhaber: Tremitas GmbH, 9020 Klagenfurth (AT)
(72) Erfinder: Zajki-Zechmeister, Tibor, 9020 Klagenfurt (AT)
(74) Vertreter: Flügel Preissner Schober Seidel
(86) Internationale Anmeldenummer: PCT/EP2019/064383
(87) Internationale Veröffentlichungsnummer: WO 2020/098980

(56) Entgegenhaltungen:
- WO-A1-2011/141734
- AT-B1- 515 976
- US-A1- 2014 073 994
- US-A1- 2018 132 756
- "Tremipen - Tremor einfach messen", Youtube, 9. April 2018 (2018-04-09), Seite 1 pp., XP054979501, Gefunden im Internet: URL:https://www.youtube.com/watch?v=goekzt ACb5s [gefunden am 2019-07-08]
- KWF | Kärntner Wirtschaftsförderungs Fonds: "Tremitas GmbH | Start:up Alpe-Adria | KWF", Youtube, 9. Mai 2019 (2019-05-09), Seite 1, XP054979505, Gefunden im Internet: URL:https://www.youtube.com/watch?v=4tYrOj QWrs0 [gefunden am 2019-07-08]
- Elke Von Rekowski: "Hightech-Stift für Tremor-Patienten - mednic.de - Tägliche News aus Medizin, Healthcare & IT", , 14. März 2018 (2018-03-14), XP055603142, Gefunden im Internet: URL:https://mednic.de/hightech-stift-fuer- tremor-patienten/7698 [gefunden am 2019-07-08]
- TIBOR ZAJKI-ZECHMEISTER: "Quantification of tremor severity with a mobile tremor pen", JOURNAL OF NEURAL TRANSMISSION, Bd. 126, Nr. 5, 5. Januar 2019 (2019-01-05), Seiten 680-680, XP055602933, Vienna ISSN: 0300-9564
- StartupTV: "Tremitas Digital Health Startups @StartupTV", Vimeo, 22 May 2018 (2018-05-22), pages 1-1, XP054980615, Retrieved from the Internet: URL:https://vimeo.com/271306104 [retrieved on 2020-06-23]
- Start:up Alpe Adria: "Startup Alpe Adria: Tremitas", Facebook, 21 July 2019 (2019-07-21), pages 1-1, XP054980616, Retrieved from the Internet: URL:https://www.facebook.com/StartupAlpeAd ria/videos/startup-alpe-adria-tremitas/497 636437654118/ [retrieved on 2020-06-23]

## Beschreibung

Die Erfindung betrifft eine Vorrichtung zum Erfassen und Auswerten von Tremor und Bradykinese im Hand- oder Armbereich eines Patienten, die bei der Benutzung in nur einer Hand des Patienten gehalten wird. Die Erfindung betrifft ferner ein Verfahren zum Erfassen, Auswerten von Tremor im Hand- oder Armbereich eines Patienten.

Als Tremor wird das unwillkürliche, sich rhythmisch wiederholende Zusammenziehen einander entgegenwirkender Muskelgruppen bezeichnet. Es gibt verschiedene Arten von Tremor, die verschiedene Ursachen haben können. So wird beispielsweise unter anderem zwischen psychogenem Tremor, meist verursacht durch psychische Störungen, essentiellem Tremor, meist verursacht durch Gendefekte oder Parkinsontremor, verursacht durch die Parkinsonkrankheit, unterschieden.

Ein weiteres Symptom während des Frühstadiums der Parkinson-Krankheit ist die Bradykinese. Bradykinese betrifft alle Muskeln im Körper eines Menschen und verursacht eine Verlangsamung der Bewegungen, insbesondere gezielte Bewegungen, parallele motorische Abläufe und feinmotorische Tätigkeiten, und kann sich bis zu einer Bewegungslosigkeit, insbesondere Akinese genannt, führen. Die Krankheit äußert sich unter anderem durch ein zunehmend kleineres und unleserlich werdendes Schriftbild.

Für einen Menschen ist es allerdings nahezu unmöglich die einzelnen Arten von Tremor nur durch menschliche Beobachtung zu unterscheiden. Um Fehldiagnosen zu vermeiden, wird versucht, anhand von sensorischen Messungen eine objektive Diagnostik, Quantifizierung oder Differenzierung zur Verfügung zu stellen, bei der die verschiedenen Arten des Tremors beziehungsweise deren Stärke erfasst werden können.

Stand der Technik ist es unter anderem, Tremor mit Hilfe einer im Wesentlichen in Form eines Schreibgeräts ausgebildeten Vorrichtung zu erfassen, wie es AT 515 976 B1 offenbart. Die gattungsgemäße Vorrichtung umfasst wenigstens einen Drucksensor im Griffbereich, wenigstens einen gyroskopischen Sensor und wenigstens einen Beschleunigungssensor und einen Sensor zum Messen des Hautwiderstands. Die mit der Vorrichtung aufgenommenen Daten können über ein Übertragungsmittel an einen Computer übergeben werden, wo sie ausgewertet werden können.

Allerdings muss der Computer mit einer entsprechenden Software ausgestattet sein und zum Auswerten der Daten von einem Fachmann mit Erfahrung in Technik und Diagnostik betrieben werden.

Weiterhin ist aus WO 2016/133621 A1 ein Handinstrument zur Messung des menschlichen Tremors bekannt, das die Form eines Löffels hat.

Eine Vorrichtung, die bei der Diagnose von Nervenleiden, wie beispielsweise der Parkinsonschen Krankheit, Anwendung findet und als Schreibgerät ausgestaltet ist, beschreibt WO 2011/141734 A1. "Tremipen - Tremor einfach messen",Youtube, https://www.youtube.com/watch?v=goekztACb5s, 9. April 2018, zeigt eine stiftförmige Vorrichtung zur Messung von Tremor.

Es ist bekannt, zum Erfassen und Auswerten von Tremor im Hand- oder Armbereich eines Patienten eine Vorrichtung zu benutzen, die in nur einer Hand des Patienten gehalten wird und die ein rohrförmiges Gehäuse, einen Beschleunigungssensor, eine Auswertungseinrichtung, ein Speichereinrichtung, eine Anzeigeeinheit und eine Aktivierungseinheit aufweist.

Der Erfindung liegt die **Aufgabe** zugrunde, eine Vorrichtung zum Erfassen, Auswerten und Differenzierung von Tremor und Bradykinese zu schaffen, mit der eine effiziente, objektive Diagnose oder Quantifizierung möglich ist.

Die Aufgabe wird durch eine Vorrichtung nach Anspruch 1 und ein Verfahren nach Anspruch 12 gelöst. Bevorzugte Ausgestaltungen der Erfindung sind Gegenstand der Ansprüche 2 bis 11 und 13 bis 15.

Das rohrförmige Gehäuse erinnert an die Form eines Schreibgerätes, wodurch es für den Patienten intuitiv zu greifen ist.

Für eine optimale Messung des Tremors wird die Vorrichtung locker in den Fingern eines Patienten gehalten. Durch das Abstützen der Vorrichtung an dem zweiten Abstützpunkt, wird das Halten der Vorrichtung an dem ersten Abstützpunkt unterstützt und beugt einem unnatürlichen Krafteinfluss durch die Griffkraft oder Verkrampfen der Finger vor.

Durch die integrierte Auswertungseinrichtung sind keine separate Auswertungseinrichtung oder sonstige weitere externe Komponenten nötig. Der Patient kann demnach auf einfache Weise selbst Messdaten aufnehmen, welche durch die Auswertungseinrichtung aufbereitet und ausgewertet werden. Ein technisch und/oder medizinisch versierter Fachmann ist somit in einem ersten Schritt nicht nötig. Für eine endgültige Diagnose oder Therapieentscheidung kann ein Arzt mit Hilfe der durch die Vorrichtung gemessenen und ausgewerteten Daten unterstützt werden.

Die Vorrichtung ist zweckmäßigerweise als sogenanntes "Handheld Device" ausgebildet, welches in einer Hand bedient werden kann.

Die Aktivierungseinrichtung dient zusätzlich als Orientierungshilfe beim Ausrichten der Vorrichtung während des Messens. Beispielsweise ist die Vorrichtung während des Messens regelmäßig derart zu halten, dass die Aktivierungsvorrichtung nach oben zeigt. Auf diese Weise ist die Vorrichtung bei jeder Benutzung gleich ausgerichtet, was die Genauigkeit der Messungen erhöht.

Erfindungsgemäß weist die Vorrichtung einen Drehratensensor auf und eignet sich damit zum Erfassen und Auswerten von Bradykinese. Der Drehratensensor ist in dem vorderen Abschnitt des Gehäuses angeordnet. Ferner ist der Drehratensensor durch die Aktivierungseinheit aktivierbar. In einer vorteilhaften Ausgestaltung dient die Batterie dazu, den Drehratensensor mit elektrischer Energie zu versorgen.

Unter Drehratensensor ist ein Sensor zu verstehen, der die Rotationgeschwindigkeit um eine definierte Achse messen kann. Der Drehratensensor kann als Gyroskop oder als kapazitiver Drehratensensor ausgebildet sein. Vorteilhaft ist der Sensor derart ausgestaltet, dass Rotationsänderungen um drei orthogonal zueinander angeordnete Achsen erfasst werden. In Kombination mit dem Beschleunigungssensor, welcher vorzugsweise die lineare Beschleunigung in drei orthogonal zueinander angeordneten Richtungen misst, kann eine drei dimensionale Kinematik errechnet werden. Somit erfasst die erfindungsgemäße Vorrichtung sechs Freiheitsgrade. Je nach Anwendungsfall kann die erfindungsgemäße Vorrichtung neben dem Beschleunigungssensor einen Drehratensensor aufweisen, der die Rotation um nur eine Achse erfasst. Auf diese Weise verfügt die erfindungsgemäße Vorrichtung zwar nur über zwei Freiheitsgrade, stellt aber eine kompakte und kostengünstige Ausgestaltung sicher.

Dadurch, dass der Drehratensensor in dem vorderen Bereich angeordnet ist, kann ein direkter Kraftfluss zwischen den Fingern und dem Drehratensensor stattfinden.

Des Weiteren besteht vorteilhafterweise die Möglichkeit, den Drehratensensor mittels der Aktivierungseinheit nur bei Bedarf zu aktivieren. Somit wird nur dann die Energie der Batterie verbraucht, die in einer vorteilhaften Ausgestaltung ebenfalls in dem Gehäuse angeordnet ist, wenn der Modus ausgewählt wird, der den Drehratensensor erfordert, wie insbesondere der Modus zum Messen der Bradykinese. Eine energiesparende Vorrichtung wird dadurch geschaffen.

Der Beschleunigungssensor ist in dem vorderen Abschnitt angeordnet. Erfindungsgemäß sind der Beschleunigungssensor und der Drehratensensor in räumlicher Nähe zueinander angeordnet. Des Weiteren sind vorzugsweise der Beschleunigungssensor und der Drehratensensor auf einer zentralen Achse, die parallel zur Längsrichtung verläuft, angeordnet. Zweckmäßigerweise bilden der Beschleunigungssensor und der Drehratensensor eine räumliche Einheit.

Da der Beschleunigungssensor ebenfalls in dem vorderen Abschnitt angeordnet ist, ist der Weg zwischen Beschleunigungssensor und/oder Drehratensensor und den Fingern einer Hand auf diese Weise möglichst gering. Somit kann ein Impuls möglichst direkt an die Sensoren hin übertragen werden. Je direkter ein Impuls übertragen wird, den der Beschleunigungssensor und/oder Drehratensensor misst, desto geringer ist die Verzögerung und verfälscht die Messung. Die Qualität der Messdaten des Beschleunigungssensors und des Drehratensensors wird somit deutlich verbessert.

Zusätzlich können in einer weiteren Ausgestaltung Messfehler mittels einer räumlichen Nähe des Drehratensensors zu dem Beschleunigungssensor reduziert werden. Somit wird es ermöglicht, die Bewegung eines definierten Punktes sehr präzise zu bestimmen.

Des Weiteren ist die zentrale Anordnung innerhalb des Gehäuses dahingehend vorteilhaft, dass unabhängig der rotierten Haltung der Vorrichtung um die Längsachse x die Sensoren vergleichbare Messwerte erfasst.

Besonders vorteilhaft hat sich gezeigt, dass eine Anordnung des Drehratensensors und des Beschleunigungssensors als eine Einheit besonders exakt Messwerte erfassen kann. Eine Einheit ist dahingehend zu verstehen, dass die einzelnen Sensoren an einer Trägereinheit befestigt sind.

Durch die Verbesserung der Qualität der Messdaten des Beschleunigungssensors werden beispielsweise Daten von weiteren Sensoren, wie insbesondere Sensoren des Hautwiderstands oder Drucksensoren überflüssig. Je weniger Sensoren vorhanden sein müssen, desto kostengünstiger, kompakter lässt sich die Vorrichtung herstellen.

Die Vorrichtung weist einen Referenzpunkt für das dritte Fingerglied von wenigstens einem Finger der Hand des Patienten auf, der an dem Gehäuse angeordnet ist.

Der Referenzpunkt dient dazu, dem Patienten eine Hilfestellung zur Handhabung der Vorrichtung auf eine intuitive Weise zu vermitteln, sodass eine reproduzierbare Messdurchführung möglich wird. Aufgrund der Anordnung des Referenzpunkts an dem Gehäuse kann eine direkte Übertragung des Impulses zu den Sensoren sichergestellt werden. Es wäre denkbar, einen solchen Referenzpunkt auch mittels einer auf dem Gehäuse zusätzlich angebrachten Vorrichtung darzustellen.

In einer kompakten Lösung ist der Referenzpunkt als eine Tippmulde vorgesehen. Somit wird ein haptisches, wahrnehmbares Merkmal geschaffen. Der Patient wird somit intuitiv darauf hingewiesen, dass beispielsweise diese Stelle bei einem Tapping-Test mit dem Zeigefinger angeschlagen werden soll. Ferner ist es denkbar, dass jeder Finger einer Hand, neben dem Zeigefinger, für einen solchen Tapping-Test verwendet werden kann.

Vorteilhaft weist die Aktivierungseinrichtung einen Druckknopf und/oder Schieberegler auf. Zweckmäßigerweise hat die Aktivierungseinrichtung wenigstens eine der folgenden Einstellpositionen:
Eine erste Einstellposition (0), die den Ausschaltzustand definiert;
eine zweite Einstellposition (T), die einen Modus zur Messung von Tremor definiert;
eine dritte Einstellposition (B), die einen Modus zur Messung von Bradykinese definiert, und
eine vierte Einstellposition (D), die einen Modus zur differentialdiagnostischen Messung definiert.

Ein Druckknopf stellt die einfachste und bekannteste Ausgestaltung einer Aktivierungseinrichtung dar. Außerdem erleichtert das haptische Feedback des Druckknopfes die Bedienbarkeit der Vorrichtung. Durch den Schieberegler wird zusätzliche eine intuitive Einstellung des Messverfahrens ermöglicht. In Kombination mit einer Beschriftung der Einstellpositionen, ist es dem Patienten leicht verständlich zu vermitteln, in welchem Modus sich die Vorrichtung befindet. Bei einer Verwendung des Schiebereglers in Kombination mit einem Druckknopf, kann eine weitere Unterauswahl des durch den Schiebregler ausgewählten Modus vorgenommen werden. Dies bedeutet, dass zuerst mittels des Schiebereglers die zu untersuchende Symptomgruppe ausgewählt wird und mittels des Druckknopfs sowie ein mehrmaliges Betätigen des Druckknopfs ein Diagnoseverfahren ausgewählt wird. Ferner wäre es denkbar, dass die Aktivierungseinrichtung illuminiert ist, insbesondere mit einem roten und/oder grünen Licht, sodass dem Benutzer zusätzlich eine Information vermittelt wird.

Vorteilhaft umfassen die Auswertungsdaten eine Amplitude des Tremors oder eine Frequenz des Tremors oder einen Energiegehalt des Tremors. In einer weiteren vorteilhaften Ausgestaltung umfassen die Auswertungsdaten ferner zumindest eines der folgenden Parameter: die Anzahl, die Intensität, die Änderung der Stärke und den Rhythmus von Tappings; wobei vorzugsweise die Auswertungsdaten ferner zumindest eines der folgenden Parameter umfassen: die Anzahl, die Intensität, die Änderung der Stärke und den Rhythmus von Änderungen der Rotationsrichtung; wobei vorzugsweise die Auswertungsdaten ferner zumindest eines der folgenden Parameter umfassen: die Anzahl, die Intensität, die Änderung der Stärke und den Rhythmus von Zieltreffern.

Die Amplitude des Tremors stellt die Beschleunigung des Tremors in g-Kraft, insbesondere milli-g dar. Beispielsweise liegt das Grundzittern eines gesunden Menschen im Bereich von 3 milli-g, während bei einem Tremor die Amplitude im Bereich von 20 bis 300 milli-g liegt. Zusätzlich werden die Frequenz des Tremors und der Energiegehalt des Tremors ausgewertet. Aufgrund der vorliegenden Daten kann ein Hinweis auf die Art des Tremors gegeben werden.

Zur Erfassung und Auswertung der Messdaten hinsichtlich Bradykinese wird zum einen eine Reihe an Taps, Pronations-Supinations-Bewegungen, die eine Änderung der Rotationsrichtung der Vorrichtung verursachen, und Zielbewegungen durchgeführt.

Unter Tap, ist ein Anschlag eines Fingers an dem Gehäuse der Vorrichtung, wodurch eine messbare Erschütterung der Vorrichtung verursacht wird, zu verstehen. Folgend auch als Tapping-Test bezeichnet. Unter einer Folge an Pronation-Supinations-Bewegungen ist eine alternierende Drehung der Hand zu verstehen, die durch eine Rotation des Unterarms erzeugt wird, so dass möglichst Elle und Speiche nach der Drehung parallel, nebeneinander liegen. Folgend auch als Pronation-Supinations-Test bezeichnet. Unter Zielbewegungen ist eine Folge an koordinierten Bewegungen zu einem Zielpunkt hin zu verstehen, wobei die Vorrichtung an zwei definierte Punkte abwechselnd hingeführt wird. Folgend wird dies als Targeting-Test bezeichnet.

Die Messdaten während eines Tapping-Tests, Pronation-Supinations-Test oder Targeting-Test umfassen die Anzahl, die Intensität, die Änderung der Stärke und den Rhythmus. Unter Anzahl ist eine dimensionslose Aufzählung an Bewegungsfolgen zu verstehen. Unter Intensität ist der durchschnittlich erfasste Messgröße der Beschleunigung in milli-g oder Rotationsgeschwindigkeit in °/sec zu verstehen. Unter Änderung der Stärke ist ein prozentueller Wert der Änderung der ersten zu der letzten Bewegungsfolge, erfasst in % zu verstehen, wobei 0 % eine gleichbleibende Amplitude und 100 % eine vollkommen unterschiedliche Amplitude beschreibt. Unter Rhythmus ist eine Reihe an Bewegungsfolgen zu verstehen, wobei die Zeit zwischen zwei folgenden Bewegungsfolgen verglichen werden. Dabei wird der Rhythmus mit einem Wert zwischen 0, vollkommen stochastisch, und 1, vollkommen gleichmäßig, in Schritten von 0,1 bewertet.

Vorteilhaft umfasst die Vorrichtung eine Schnittstelle, vorzugsweise eine drahtlose Schnittstelle, zum Austauschen der Auswertungsdaten, zum Austauschen der Messdaten und/oder Rohdaten und/oder zum Austauschen von Einstellungen der Aktivierungseinrichtung mit einem externen Gerät.

Die ausgewerteten Daten können auf der Vorrichtung gespeichert werden. Zweckmäßigerweise finden die Messung und Auswertung des Tremors über einen längeren Zeitraum, beispielsweise mehrere Wochen, statt. Die endgültige Diagnose und Art der Behandlung soll jedoch abschließend von einer medizinischen Fachkraft diagnostiziert werden. Durch die Schnittstelle, können die gemessenen und/oder ausgewerteten Daten an die medizinische Fachkraft übersendet werden, welche die Daten erneut evaluieren kann, was zu einer optimalen Diagnose und folglich zu einer optimalen Behandlung führt. Damit die Übertragung möglichst einfach und fließend in den Arbeitsalltag eines Arztes integrieren lässt, ist in einer vorteilhaften Ausgestaltung eine drahtlose Verbindung vorgesehen, die eine Vorrichtung mit einem Kabel zur Datenübertragung ersetzt.

Ferner kann ein externes Gerät, ein mobiles Telefon oder ein Gerät eines Arztes umfassen. Mittels des Geräts des Arztes kann dieser eine Diagnose durchführen oder die erfassten Messdaten weiter verarbeiten, beziehungsweise in einer für den Patienten angelegten Patientenakte archivieren. Vorteilhaft ist es, dass durch die Übertragung der Messdaten, während der Messung, parallel die Messwerte angezeigt werden können. Des Weiteren kann eine Anpassung der Einstellung der Vorrichtung vorgenommen werden. Eine Kommunikation in beide Richtungen ermöglicht dies. Zweckmäßigerweise wird hierfür ein W-LAN oder Bluetooth Standard verwendet.

Erfindungsgemäß umfasst die Vorrichtung eine Signaleinheit zum Ausgeben von zumindest akustischen oder haptisch wahrnehmbaren Signalen. Die Signaleinheit kann als ein Lautsprecher oder ein Vibrationsmotor ausgestaltet sein.

Um die Bedienbarkeit der Vorrichtung weiter zu verbessern und sie insbesondere intuitiver zu gestalten, gibt die Vorrichtung akustische oder haptisch wahrnehmbare Signale aus. Akustisches Feedback gibt dem Patienten eine Bestätigung für die korrekte Bedienung der Vorrichtung. Beispielsweise gibt die Signaleinheit, insbesondere ein Lautsprecher, ein akustisches Signal aus, wenn die Vorrichtung aktiviert und/oder deaktiviert wird, wenn die Messung beginnt und/oder endet und/oder wenn die Auswertung der gemessenen Daten beginnt und/oder endet. Denkbar (jedoch nicht beansprucht) ist es, dass die Signaleinheit ein optisch wahrnehmbares Signal abgibt.

Zusätzlich kann die Signaleinheit eine bestimmte Reihenfolge von Signalen, insbesondere akustische, ausgeben, um beispielsweise Fehlercodes zu signalisieren, wie zum Beispiel einen niedrigen Ladezustand der Batterie, ein Fehler in der Messung oder Auswertung.

Vorteilhaft weist das Gehäuse eine Länge zwischen 10 cm und 20 cm, insbesondere zwischen 14 cm und 17 cm, in der Längsrichtung auf und vorzugsweise einen Durchmesser zwischen 10 mm und 40 mm, insbesondere zwischen 15 mm und 30 mm auf, wobei vorzugsweise der vordere Abschnitt zwischen 10% und 60% der Länge aufweist, der mittlere Abschnitt zwischen 10% und 60% der Länge aufweist und der hintere Abschnitt zwischen 10% und 60% der Länge aufweist, wobei ferner vorzugsweise das Gehäuse zumindest in dem vorderen Abschnitt sich in der Längsrichtung konisch verjüngend ausgestaltet ist. Die Abmessungen des Gehäuses der Vorrichtung sind entsprechend der Anwendung optimiert. Die Länge des Gehäuses ist mindestens so lang, dass es bei üblichen Handgrößen möglich ist, das dritte Fingerglied (Phalanx distalis) von wenigstens einem Finger der Hand des Patienten an dem ersten Abstützpunkt abzustützen und der zweite Abstützpunkt in dem Zwischenraum zwischen Daumen und Zeigefinger der Hand des Patienten abstützt. Ist die Länge des Gehäuses allerdings zu groß, verlagert sich der Schwerpunkt der Vorrichtung weg von dem ersten und zweiten Abstützpunkt. Dieser Effekt hat durch seine Hebelwirkung zu dem ersten und zweiten Abstützpunkt eine erhebliche negative Auswirkung auf die Qualität der Messung. Am bevorzugtesten hat sich eine Länge von 16 cm herausgestellt.

Das Gehäuse weist einen Durchmesser auf, der für eine übliche Fingergröße eines Patienten gut greifbar ist. Allerdings haben Patienten, welche die Vorrichtung benutzen, häufig feinmotorische Einschränkungen. Der Durchmesser des Gehäuses ist deshalb groß genug, dass Patienten mit feinmotorischen Einschränkungen das Gehäuse sicher greifen können.

Das Gehäuse der Vorrichtung verjüngt sich in dem vorderen Abschnitt in der Längsrichtung. Die Form des Gehäuses erinnert dadurch der Form eines üblichen Schreibgeräts und erlaubt es dem Patienten intuitiv wahrzunehmen, wie die Vorrichtung zu halten ist. Am bevorzugtesten hat sich ein Durchmesser von 20 mm herausgestellt.

Der vordere, mittlere und hintere Abschnitt muss nicht gleichmäßig auf die Länge der Vorrichtung verteilt sein. Je nach Ausgestaltung kann jeder der Abschnitte 10% bis 60% der Länge der Vorrichtung aufweisen.

Vorteilhaft umfasst die Vorrichtung wenigstens eine Platine, auf welcher der Beschleunigungssensor, der Drehratensensor, die Auswertungseinrichtung, die Speichereinrichtung, die Anzeigeeinrichtung, die Aktivierungseinrichtung, die Batterie, die drahtlose Schnittstelle und/oder die Signaleinheit angeordnet sind, wobei vorzugsweise die Platine formschlüssig und/oder kraftschlüssig in dem Gehäuse gehalten ist und wobei vorzugsweise das Gehäuse wenigstens eine Führungsschiene zum Aufnehmen der Platine aufweist. Ferner sind vorzugsweise der Beschleunigungssensor und der Drehratensensor auf der Platine nebeneinander angeordnet.

Die Anordnung des Beschleunigungssensors, des Drehratensensors der Auswertungseinrichtung, der Speichereinrichtung und/oder der Batterie auf einer Platine vereinfacht die Kommunikation unter den einzelnen Elementen. Damit eine möglichst optimale Messung durch den Beschleunigungssensor oder den Drehratensensor sichergestellt wird, ist die Platine formschlüssig und/oder kraftschlüssig in dem Gehäuse gehalten. Eine besondere bevorzugte Methode ist das Vorsehen einer Führungsschiene zum Aufnehmen der Platine in dem Gehäuse. Nach dem Herstellen des Gehäuses kann die Platine über die Führungsschiene einfach in das Gehäuse geschoben werden. Anschließend wird die Platine mit der Führungsschiene formschlüssig und/oder kraftschlüssig verbunden, um einen sicheren Halt zu gewährleisten. Es wäre denkbar in einer weiteren Ausgestaltung den Drehratensensor und den Beschleunigungssensor auf zwei separate Platinen anzuordnen.

Vorteilhaft sind die Anzeigeeinrichtung in dem mittleren Abschnitt und vorzugsweise die Batterie in dem hinteren Abschnitt angeordnet, wobei ferner vorzugsweise das Gehäuse mit wenigstens einer Öffnung zur Aufnahme der Anzeigeeinrichtung, der Aktivierungseinrichtung und/oder des Lautsprechers versehen ist.

Zweckmäßigerweise kann auch die Anzeigeeinrichtung, die Aktivierungseinrichtung und/oder der Lautsprecher direkt an dem Gehäuse angeordnet sein und durch eine Verbindung, insbesondere eine Kabelverbindung, mit der Platine im inneren der Vorrichtung verbunden sein. In diesem Fall ist für das jeweilige Element keine Öffnung im Gehäuse notwendig.

Die Anzeigeeinrichtung kann zweckmäßigerweise auch während des Messvorgangs einsehbar sein. Der mittlere Abschnitt der Vorrichtung ist während des Messvorgangs kaum durch die Hand und nicht durch Finger des Patienten verdeckt. Das Anordnen der Anzeigeeinrichtung im mittleren Abschnitt ermöglicht es dem Patienten somit auch während des Messvorgangs die darauf angezeigten Daten einzusehen. Obwohl es naheliegt, die Aktivierungseinrichtung und die Anzeigeeinrichtung nebeneinander anzuordnen, kann die Anzeigeeinrichtung auch auf dem Gehäuse versetzt angeordnet werden, sodass bei Bedienung der Vorrichtung während des Messvorgangs die Aktivierungseinrichtung zur Ausrichtung der Vorrichtung nach oben zeigt und die Anzeigeeinrichtung derart versetzt ist, dass der Patient diese in einem geradem Winkel einsehen kann.

Die Batterie weist ein hohes Gewicht im Vergleich zu den weiteren Komponenten der Vorrichtung auf. Deshalb ist es vorteilhaft die Batterie in dem hinteren Abschnitt anzuordnen. Dort unterstützt das Gewicht der Batterie das Abstützen der Vorrichtung am zweiten Abstützpunkt an dem Zwischenraum zwischen Daumen und Zeigefinger der Hand des Patienten.

Vorteilhaft weist das Gehäuse einen abnehmbaren Deckel zum Einlegen der Batterie auf, wobei zweckmäßigerweise die Platine durch den Deckel in dem Gehäuse eingespannt ist.

Vorteilhaft besteht das Gehäuse aus Kunststoff, insbesondere Polykarbonat.Kunststoff ermöglicht es dem Gehäuse eine bevorzugte Mischung aus Stabilität und Gewicht zu erreichen. Ein bevorzugtes Gewicht des Gehäuses beträgt 35 Gramm.

Vorteilhafterweise befindet sich ein Schwerpunkt in dem mittleren Abschnitt.Eine verbesserte Handhabung der Vorrichtung wird somit geschaffen. Dabei befindet sich der Schwerpunkt der Vorrichtung in einer vorteilhaften Ausgestaltung in der Nähe des zweiten Abstützpunkts. Die Haltung der stiftähnlichen Vorrichtung liegt somit besonders vorteilhaft und ausbalanciert in der Hand des Patienten. Somit ruht die Vorrichtung auf dem zweiten Abstützpunkt und der Patient benötigt weniger Kraft in den Fingern um die Vorrichtung fest zu halten.

Vorteilhaft weist der Beschleunigungssensor und/oder der Drehratensensor eine Abtastrate von jeweils mindestens 50 Hz, oder vorzugsweise mindestens 100 Hz oder am bevorzugtesten mindestens 200 Hz auf.

Mittels einer Abtastrate von mindestens 50 Hz ist es möglich, den Tapping-Tests, den Pronation- und Supinations- Tests und den Targeting -Tests durchzuführen. Mittels den dabei erfassten Daten wird eine Wahrscheinlichkeit über eine mögliche Erkrankung an Bradykinese diagnostiziert. Eine Abtastrate von mindestens 100 Hz des Beschleunigungssensors ermöglicht eine Diagnose über die Wahrscheinlichkeit einer Erkrankung an Tremor zu diagnostizieren. Eine Abtastrate von mindestens 200 Hz hingegen ermöglicht die Durchführung der Differentialdiagnose von Tremor. Die Abtastrate ist dahingehend ein wesentlicher Bestandteil, dass der Aliasing-Effekt reduziert oder sogar ausgeschlossen werden kann.

In einer Vorteilhaften Ausgestaltung weist die Vorrichtung eine zusätzliche Testeinheit zum Durchführen eines Targeting-Tests auf, welche wenigstens zwei Anschlagelemente aufweist, die in einem vorgegebenen Abstand zueinander angeordnet sind; wobei die Anschlagelemente jeweils eine Kontaktfläche aufweisen, die geeignet ist, durch den vorderen Abschnitt bei einem Anschlag der Vorrichtung an das Anschlagelement kontaktiert zu werden.

Mittels der zusätzlichen Einheit wird dem Patienten eine Hilfestellung in der Durchführung eines Targeting-Tests gegeben. Dabei wird es ermöglicht, dem Patienten einen gleichbleibenden, vergleichbaren Bewegungsablauf vorzugeben, indem sich das zu treffende Ziel in einem vorgegeben, konstantem Abstand zueinander befindet. Die Anschlagelemente können ferner dämpfende Elemente aufweisen und würden somit einen weicheren Anschlag schaffen, der zum einen schonend für das Gerät und zugleich für die Gelenke des Patienten ist.

Ferner werden gleichbleibende Rahmenbedingungen für alle Patienten geschaffen, sodass diese untereinander verglichen werden können.

In einer weiteren Vorteilhaften Ausgestaltung weist die Testeinheit einen Zielpunkt auf, der sich auf der Kontaktfläche befindet. Zweckmäßigerweise ist der Zielpunkt im Wesentlichen in der Mitte der Kontaktfläche angeordnet.

Der Zielpunkt ermöglicht eine leicht erkennbare und intuitive Orientierung für den Patienten. Der Zielpunkt definiert einen Bereich auf der Kontaktfläche durch eine Kennzeichnung. Diese Kennzeichnung kann in einer möglichen Ausgestaltung ein bunter Kreis sein oder eine Kerbe. Dadurch dass der Zielpunkt im Wesentlichen in der Mitte der Kontaktfläche ist, wird der Patient mit einer höheren Wahrscheinlichkeit die Kontaktfläche treffen. Durch eine solche Ausführungsform kann die Durchführung eines Targeting-Tests dem Patienten leicht und verständlich vermittelt werden.

Vorteilhaft ist der Abstand zwischen den Anschlagelementen verstellbar; wobei vorzugsweise der Abstand zwischen den Anschlagelementen zwischen 5 cm und 100 cm, bevorzugt zwischen 10 cm und 50 cm, am bevorzugtesten zwischen 15 cm und 25 cm beträgt.

Dadurch dass der Abstand zwischen den Anschlagelementen verstellbar ist, ist eine individuelle Anpassung möglich. Der Abstand wird zwischen den Zielpunkten gemessen. Beispielsweise kann für ein Kind ein anderer Abstand eingestellt werden als für einen Erwachsenen oder für einen Patienten der nur beschränkt Bewegungen durchführen kann. Generell hat sich jedoch gezeigt, dass ein Abstand von ca. 20 cm besonders vorteilhaft für die Durchführung eines Targeting-Tests ist.

Vorteilhaft weist die Testeinheit einen Verbindungssteg auf, der die Anschlagelemente miteinander verbindet; wobei vorzugsweise der Abstand zwischen den Anschlagelementen durch den Verbindungssteg verstellbar ist.

Mittels des Verbindungsstegs kann während eines Targeting-Tests ein konstanter und gleichbleibender Abstand gewährleistet werden. Dadurch, dass dieser verstellbar ist, kann eine einfachere und kompaktere Handhabung der Einheit geschaffen werden, aber auch eine Anpassung an die Bedürfnisse unterschiedlicher Patienten. Die Verstellung kann teleskopartig erfolgen.

Vorteilhaft weist die Testeinheit eine Erfassungseinheit auf, durch die zumindest einer der folgenden Parameter erfasst wird: die Anzahl an Anschlägen, die Zeit zwischen zwei Anschlägen und die Gesamtdauer des Tests; wobei vorzugsweise der erfasste Parameter mittels eines Displays anzeigbar ist; wobei vorzugsweise das Display an dem Verbindungssteg angeordnet ist.

Die Erfassungseinheit ist in einer weiteren voreilhaften Ausgestaltung mit der Vorrichtung mittels einer drahtlosen Verbindung koppelbar. Die Erfassungseinheit dient dazu, Vorgänge und Bewegungsfolgen zu erfassen, die anschließend mittels des Displays angezeigt werden kann. Dadurch wird dem Patienten die erfasste Information direkt vermittelt.

Vorteilhaft weist die Testeinheit eine Signaleinheit auf, die geeignet ist, bei einem Anschlag der Vorrichtung an einem der Anschlagelemente ein Signal zu erzeugen; wobei vorzugsweise die Signaleinheit als Leuchtelement insbesondere als LED, oder als Lautsprecher ausgestaltet ist.

Die Signaleinheit kann insbesondere ein Lautsprecher, ein Leuchtelement oder ein Vibrationsmotor umfassen. Mittels der Signaleinheit kann dem Patienten das Feedback vermittelt werden, ob dieser mit der Vorrichtung einen Kontakt mit der Einheit hergestellt hat. Dadurch kann dem Patienten angezeigt werden, ob dieser mit der Vorrichtung ausreichend weit die vorgegebene Bewegung durchgeführt hat und kann somit dem Patienten eine Hilfestellung zur Selbstkontrolle geben.

Die Erfindung beinhaltet ein Verfahren nach Anspruch 12.

Das Verfahren zeichnet sich durch seine intuitive Führung des Patienten durch die einzelnen Verfahrensschritte aus. Zudem bietet das Verfahren eine kompakte, zeiteffiziente Möglichkeit den Tremor eines Patienten zu messen und autark auszuwerten. Für den Patienten entspricht die Komplexität der Anwendung der Vorrichtung und des Verfahrens in etwa der Komplexität der Anwendung eines üblichen Fieberthermometers.

Das Verfahren ermöglicht eine autarke Messung und Auswertung des Tremors eines Patienten. Externe Komponenten sind somit nicht nötig.

Außerdem wird der Patient nicht gezwungen die Vorrichtung während der Messung zu bewegen. Ein Zeichnen von Formen, wie es bei bekannten Messverfahren eines Tremors üblich ist, entfällt. Der Patient muss während der Messdauer die Vorrichtung lediglich entspannt und unbelastet in der vorgesehenen Position halten.

Vorteilhaft beträgt die Messdauer weniger als 60 Sekunden, insbesondere weniger als 40 Sekunden. In der Praxis hat sich eine Messdauer von ca. 30 Sekunden als besonders geeignet herausgestellt. Vorzugsweise beträgt die Auswertungsdauer weniger als 10 Sekunden. Die Anzeigedauer beträgt erfindungsgemäß wenigstens 20 Sekunden, insbesondere mehr als 45 Sekunden. Eine Anzeigedauer von ca. 30 Sekunden hat sich jedoch in vielen Fällen als besonders praxisgerecht herausgestellt.

Der gesamte Mess-, Auswerte- und Anzeigevorgang hält somit einen überschaubaren zeitlichen Rahmen ein. Die Messdauer und die Auswertungsdauer sollen so kurz wie möglich sein, jedoch ist eine gewisse Messdauer für ein belastbares Messergebnis nötig, da die Auswertungsdauer abhängig von der Rechenleistung der Auswertungseinrichtung und der Komplexität der auszuwertenden Daten ist. Die Anzeigedauer kann je nach Präferenz eingestellt werden.

Ein (nicht under die Ansprüche fallendes) Verfahren zum Erfassen und Auswerten von Bradykinese im Hand- oder Armbereich eines Patienten mit Hilfe einer der oben genannten Vorrichtung umfasst die Schritte.
Aktivieren der Vorrichtung mittels der Aktivierungseinrichtung zum Messen von Bradykinese;
Halten der Vorrichtung mit einem vorgegebenen Griff der Hand des Patienten;
Ausführen einer ersten Bewegungsfolge;
Messen von Messdaten über die Bradykinese mittels des Beschleunigungssensors und des Drehratensensors während einer bestimmten Messdauer;
Auswerten der Messdaten und Bereitstellen von Auswertungsdaten mittels der Auswertungseinrichtung während einer vorbestimmten Auswertungsdauer;
Speichern der Messdaten oder der Auswertungsdaten mittels der Speichereinrichtung;
Anzeigen der Auswertungsdaten mittels der Anzeigeeinrichtung für eine vorbestimmte Anzeigedauer und
Deaktivieren der Vorrichtung nach Ablauf der Anzeigedauer.

Vorteilhaft weist das Verfahren eine erste Bewegungsfolge auf, bei der alternierende Pronations- und Supinations-Bewegungen ausgeführt werden; wobei vorzugsweise hierbei die Vorrichtung mit der Hand des Patienten in einer ersten Haltung des Griffs fest ergriffen wird.

Die erste Haltung der Hand bildet einen ersten Griff, der eine faustartige Umschließung der Vorrichtung umfasst. Dadurch wird eine Haltung der Vorrichtung, die im Wesentlichen im Lot zum Unterarm ist, geschaffen. Die rotierende Bewegung der Messvorrichtung kann dadurch besonders gut erfasst werden. Denkbar wäre es auch einen anderen Griff zu verwenden, wobei die Vorrichtung fest in einer konstanten Position zur Hand positioniert wird. Dabei wird die Pronation-Supinations Bewegung erfasst.

Vorteilhaft weist das Verfahren eine zweite Bewegungsfolge auf, bei der Tippbewegungen eines Fingers der Hand des Patienten auf den Referenzpunkt ausgeführt werden; wobei vorzugsweise hierbei die Vorrichtung mit der Hand des Patienten in einer zweiten Haltung des Griffs so ergriffen wird, dass das dritte Fingerglied von wenigstens einem Finger der Hand des Patienten an dem wenigstens einen ersten Abstützpunkt, den das Gehäuse bildet, und der hintere Abschnitt des Gehäuses in dem Zwischenraum zwischen Daumen und Zeigefinger der Hand des Patienten an dem wenigstens einen zweiten Abstützpunkt, den das Gehäuse bildet, abgestützt wird.

Die zweite Haltung einer Hand bildet einen zweiten Griff, welcher die Vorrichtung derart hält, dass der Tapping-Test durchgeführt werden kann. Hierbei wird die Vorrichtung durch einen Finger, insbesondere dem Zeigefinger, erschüttert. Dadurch kann eine Beschleunigung, die dadurch verursacht wird, gemessen werden.

In vorteilhafter Weise weist das Verfahren eine dritte Bewegungsfolge auf, bei der Hin- und Herbewegungen der Hand des Patienten zwischen zwei vorgegebenen Zielpunkten ausgeführt werden; wobei vorzugsweise hierbei die Vorrichtung mit der Hand des Patienten in einer dritten Haltung des Griffs fest ergriffen wird. Durch den dritten Griff der Vorrichtung kann der Patient den vorderen Abschnitt der Vorrichtung koordiniert führen.

Vorteilhaft stehen dem Verfahren Zielpunkte zur Verfügung, die durch eine oben beschriebene Testeinheit bereitgestellt werden.

Dem Patienten kann durch eine solche Testeinheit das Verfahren besonders intuitiv und leicht vermittelt werden.

Vorteilhaft beträgt die Messdauer weniger als 60 Sekunden, insbesondere weniger als 30 Sekunden, bevorzugt ca. 10 Sekunden, wobei vorzugsweise die Auswertungsdauer weniger als 10 Sekunden beträgt, und wobei vorzugsweise die Anzeigedauer wenigstens 20 Sekunden, insbesondere mehr als 45 Sekunden, beträgt.

Die benötigte Messdauer für das Verfahren zeichnet sich von einer besonders kurzen Messdauer aus, jedoch führt eine längere Messdauer zu einem genaueren Ergebnis, da mehr Daten über einen längeren Zeitraum zur Auswertung zur Verfügung stehen.

Ein nicht unter die Ansprüche fallendes Verfahren zum Erfassen und Auswerten von Tremor im Hand- oder Armbereich eines Patienten mittels Differentialdiagnose und mit Hilfe von einer Vorrichtung der oben genannten Art, umfassend die folgenden Schritte:
Aktivieren der Vorrichtung mittels der Aktivierungseinrichtung zum Erfassen von Tremor mittels der Differentialdiagnose;
Halten der Vorrichtung derart, dass das dritte Fingerglied (Phalanx distalis) von wenigstens einem Finger der Hand des Patienten an dem wenigstens einen ersten Abstützpunkt, den das Gehäuse bildet, und der hintere Abschnitt des Gehäuses in dem Zwischenraum zwischen Daumen und Zeigefinger der Hand des Patienten an dem wenigstens einen zweiten Abstützpunkt, den das Gehäuse bildet, abgestützt wird;
Messen von Messdaten über den Tremor mittels des Beschleunigungssensors während einer bestimmten Messdauer;
Auswerten der Messdaten und Bereitstellen von Auswertungsdaten mittels der Auswertungseinrichtung während einer vorbestimmten Auswertungsdauer;
Speichern der Messdaten oder der Auswertungsdaten mittels der Speichereinrichtung;
Anzeigen der Auswertungsdaten mittels der Anzeigeeinrichtung für eine vorbestimmte Anzeigedauer und
Deaktivieren der Vorrichtung nach Ablauf der Anzeigedauer.

Das Verfahren mittels Differentialdiagnose zeichnet sich durch die hohe Abtastrate aus. Dadurch kann mittels des Verfahrens besonders gut eine Diagnose dahingehend durchgeführt werden, dass zwischen verschiedenen Arten des Tremors unterschieden werden kann.

Vorteilhaft beträgt die Messdauer mindestens 60 Sekunden, wobei zweckmäßigerweise die Auswertungsdauer weniger als 10 Sekunden beträgt, und wobei zweckmäßigerweise die Anzeigedauer wenigstens 20 Sekunden, insbesondere mehr als 45 Sekunden, beträgt.

Die verlängerte Messdauer ermöglicht es eine genauere Auswertung durchzuführen die auf einem umfassenderen Datensatz beruht.

Vorteilhaft werden die Messdaten durch die Auswertungseinrichtung gefiltert und vorzugsweise von dem Zeitbereich in den Frequenzbereich transformiert.

Die Auswertungseinrichtung filtert die Messreihe dahingehend, dass unnötige oder störende Frequenzen in der Messreihe ausgeschlossen werden. Messrauschen und Frequenzen die nicht relevant sind werden dadurch nicht berücksichtigt. Eine geglättete Kurve kann dadurch dargestellt werden. Die restlichen relevanten Daten werden dann direkt ausgewertet oder abhängig von den auszuwertenden Auswertungsdaten zuerst von dem Zeitbereich in den Frequenzbereich transformiert, um effizienter weiterverarbeitet zu werden.

Erfindungsgemäß wird durch die Signaleinheit zu Beginn der Messdauer und/oder zu Beginn Auswertungsdauer und/oder zu Beginn der Anzeigedauer wenigstens ein Signal abgegeben, wobei vorzugsweise wenigstens ein weiteres Signal am Ende der Messdauer und/oder am Ende der Auswertungsdauer und/oder am Ende der Anzeigedauer abgegeben wird, wobei vorzugsweise das Signal ein akustisch, visuell oder haptisch wahrnehmbares Signal ist.

Die Signaleinheit vermittelt dem Patienten durch das Feedback in Form eines Signals in welchem Zustand es sich befindet. Die Signaleinheit kann insbesondere einen Lautsprecher, eine LED oder ein Vibrationsmotor umfassen.

Ein Ausführungsbeispiel wird im Folgenden anhand schematischer Zeichnungen näher erläutert. Darin zeigt:
Fig. 1 eine Draufsicht auf die Vorrichtung im geöffneten Querschnitt;
Fig. 2 eine Draufsicht auf die Vorrichtung;
Fig. 3 eine Perspektivansicht der Vorrichtung mit geöffnetem Deckel;
Fig. 4 die Vorrichtung gehalten in der Hand eines Patienten während des Messvorgangs des Tremor Tests;
Fig. 5a und 5b eine beispielhafte Bewegungsabfolge während eines Pronation- Supinations-Tests;
Fig. 6a und 6b eine beispielhafte Bewegungsabfolge während eines Tapping-Tests;
Fig. 7a, 7b und 7c eine beispielhafte Bewegungsabfolge eines Targeting-Tests und
Fig. 8a und 8b jeweils ein Flussdiagramm eines Mess-/Auswertungsvorgangs der Vorrichtung.

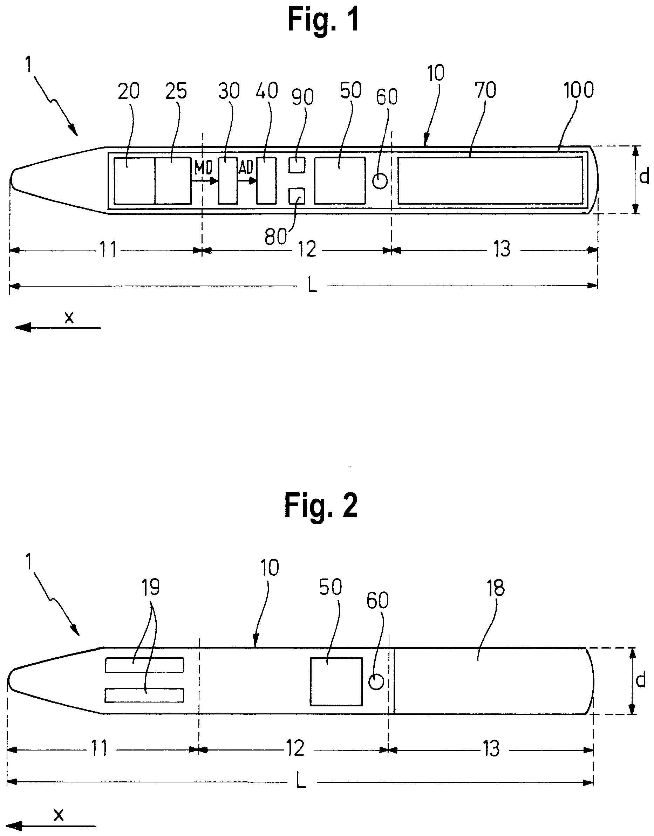

Fig. 1 zeigt eine Draufsicht der Vorrichtung 1 zum Erfassen und Auswerten von Tremor im geöffneten Querschnitt, umfassend das Gehäuse 10. Die Vorrichtung 1 ist aufgeteilt in einen vorderen Abschnitt 11, einen mittleren Abschnitt 12 und einen hinteren Abschnitt 13. Auf einer Platine 100, die sich innerhalb des vorderen Abschnitts 11, des mittleren Abschnitts 12 und des hinteren Abschnitts 13 erstreckt, sind ein Beschleunigungssensor 20, ein Drehratensensor 25 eine Auswertungseinrichtung 30, eine Speichereinrichtung 40, eine Anzeigeeinrichtung 50, eine Aktivierungseinrichtung 60 in Form eines Druckknopfes, eine Batterie 70, eine drahtlose Schnittstelle 80 und eine Signaleinheit 90 angeordnet. Die jeweiligen Elemente werden durch eine Steuereinrichtung (nicht gezeigt) gesteuert. Die Auswertungseinrichtung 30 und die Speichereinrichtung 40 können je nach Anwendungsfall auch in einem gemeinsamen Chip verbaut sein.

Der Beschleunigungssensor 20 und der Drehratensensor 25 ist derart auf der Platine 100 platziert, dass er im vorderen Abschnitt 11 der Vorrichtung 1 angeordnet ist. Der Drehratensensor 25 ist vorteilhaft um den Beschleunigungssensor 20 herum angeordnet.

Während des Messens erfasst der Beschleunigungssensor 20 Messdaten MD über den Tremor, welche von der Auswertungseinrichtung 30 autark ausgewertet werden. Die Auswertungseinrichtung 30 stellt dann Auswertungsdaten AD bereit. Die Auswertungsdaten AD können daraufhin in der Speichereinrichtung 40 gespeichert werden.

Im hinteren Abschnitt 13 der Vorrichtung 1 ist die Batterie 70 angeordnet, welche die Platine 100 und alle darauf befindlichen weiteren Elemente mit elektrischer Energie versorgt.

Das Gehäuse 10 weist eine Länge L und einen Durchmesser d auf. Die Länge L beträgt 16 cm und der Durchmesser d beträgt 20 mm. Im vorderen Abschnitt 13 verjüngt sich das Gehäuse 10 in Längsrichtung x konisch.

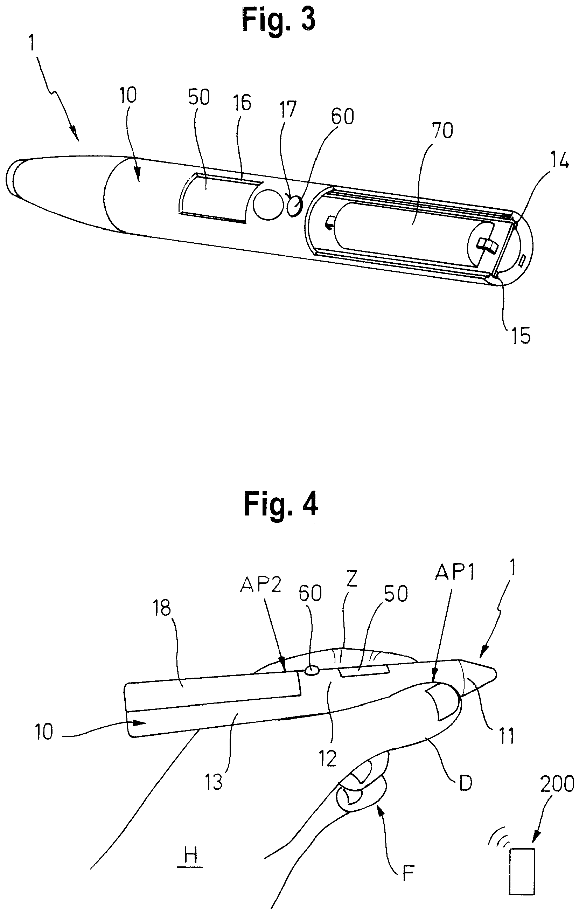

Fig. 2 zeigt eine Draufsicht der Vorrichtung 1 in geschlossenem Zustand. Ein Deckel 18 zum Öffnen und Schließen des Gehäuses 10 ist in dem hinteren Abschnitt 13 angeordnet. Die Aktivierungseinrichtung 60 und die Anzeigeeinrichtung 50 sind, wie Fig. 3 zu erkennen gibt, in Öffnungen 16, 17 des Gehäuses 10 im mittleren Abschnitt 12 angeordnet und somit von außen sichtbar. Im vorderen Abschnitt 11 weist das Gehäuse Vertiefungen 19 auf, welche als Anlageflächen für die Finger des Patienten dienen sollen.

Fig. 3 zeigt eine Perspektivansicht der Vorrichtung 1, wobei der Deckel 18 zum Öffnen der Vorrichtung 1 entfernt ist. Das Gehäuse 10 weist Führungsschienen 14, 15 auf, in welchen die Platine 100 in das Gehäuse 10 eingeschoben ist. Zum Befestigen der Platine 100 an dem Gehäuse 10 ist die Platine 100 stoffschlüssig, beispielsweise durch Verkleben, und/oder kraftschlüssig, etwa vorteilhafterweise durch ein Einklemmen mittels des Deckels 18, an dem Gehäuse 10 befestigt.

Fig. 4 zeigt die Vorrichtung 1 gehalten in der Hand H eines Patienten während des Messvorgangs. Der Patient greift die Vorrichtung 1 mit den Fingern seiner Hand, sodass die Vorrichtung 1 mit dem vorderen Abschnitt 11 an dem dritten Fingerglied (Phalanx distalis) von wenigstens einem Finger F der Hand H des Patienten an dem ersten Abstützpunkt AP1 abgestützt ist. Dabei wird die Vorrichtung 1 in dem hinteren Abschnitt 13 an einem zweiten Abstützpunkt AP2 in dem Zwischenraum zwischen Daumen D und Zeigefinger Z der Hand H des Patienten abgestützt. Diese Position wird entsprechend Messposition genannt.

Nachfolgend werden Ausführungsformen der Vorrichtung beschrieben, wobei zu deren Beschreibung die bereits zuvor verwendeten Bezugszeichen für gleiche oder funktionsgleiche Teile verwendet werden.

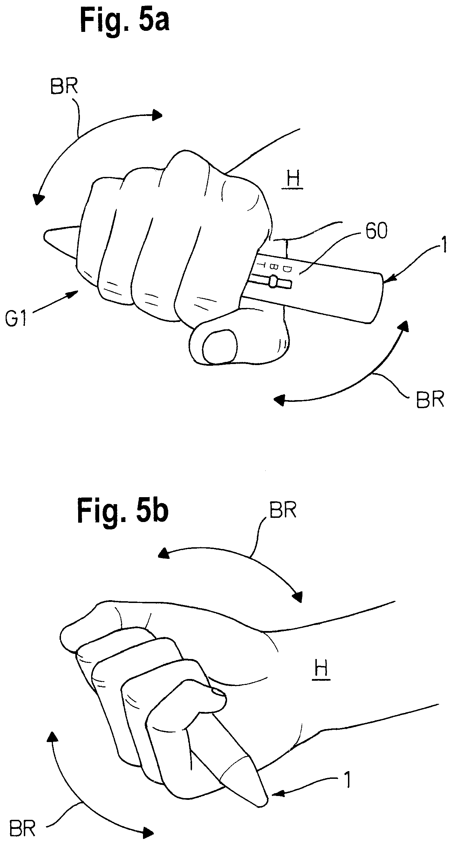

Fig. 5a und 5b zeigen einen beispielhaften Bewegungsablauf einer Vorrichtung zum Messen und Auswerten von Bradykinese während eines Pronation-Supinations-Tests. Dabei wird die Vorrichtung 1 in einer Hand H des Patienten mittels eines ersten Griffs G1 derart gehalten, dass sich die Vorrichtung 1 möglichst im Lot zum Unterarm befindet.

Dadurch kann eine optimale Erfassung der alternierenden Rotationsbewegung ermöglicht werden. Die Rotationsbewegung wird durch eine abwechselnde Drehung der Hand H durch Rotation des Unterarms erzeugt. Am Ende der Drehung liegt die Elle und Speiche möglichst parallel nebeneinander. Eine solche Bewegung kommt einer Rotation der Handfläche von 180° nahe. Jedoch ist die Drehung nicht auf 180° beschränkt, sondern kann auch deutlich geringer ausfallen. Da mit zunehmendem Alter des Patienten, die Beweglichkeit merklich eingeschränkt sein kann. Es ist somit von der maximalen Rotation auszugehen, die der Patient durchführen kann. Der Patient wiederholt diese Bewegungsfolge über eine definierte Messdauer T1_{B}.

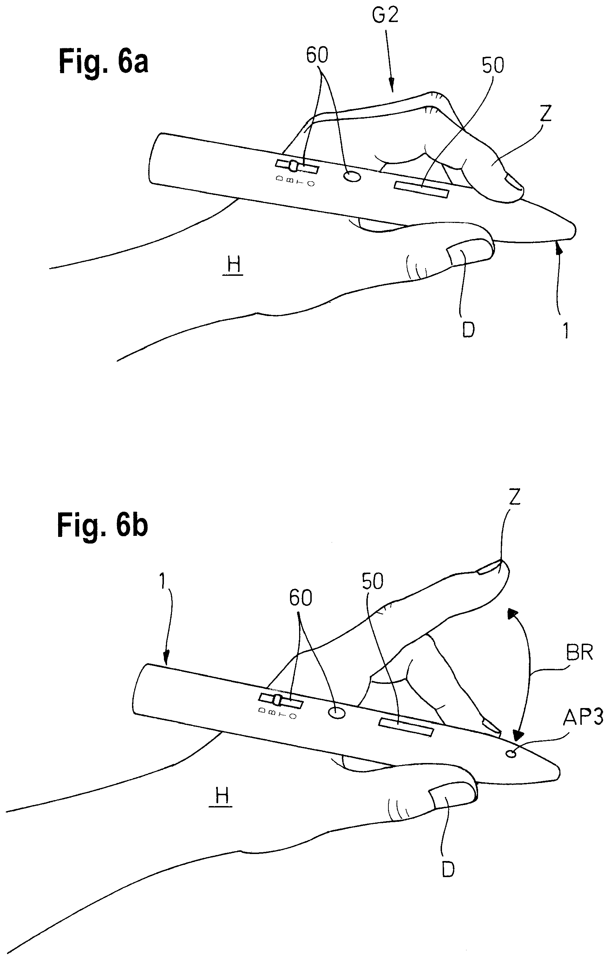

Fig. 6a und 6b zeigen einen beispielhaften Bewegungsablauf eines Tapping-Tests. Die Vorrichtung 1 wird von der Hand H eines Patienten in einem zweiten Griff G2 gehalten. Der Zeigefinger Z, der anfangs auf dem Referenzpunkt AP3 ruht, wird anschließend angehoben und gestreckt, wie es in Fig. 6b dargestellt ist, und folgend wieder zu dem Referenzpunkt AP3 zurück geführt, bis der Zeigefinger Z an dem Referenzpunkt AP3 anschlägt. Durch den Anschlag wird eine Erschütterung der Vorrichtung 1 erzeugt. Diese Bewegungsfolge wird über eine definierte Messdauer T1_{T} durchgeführt.

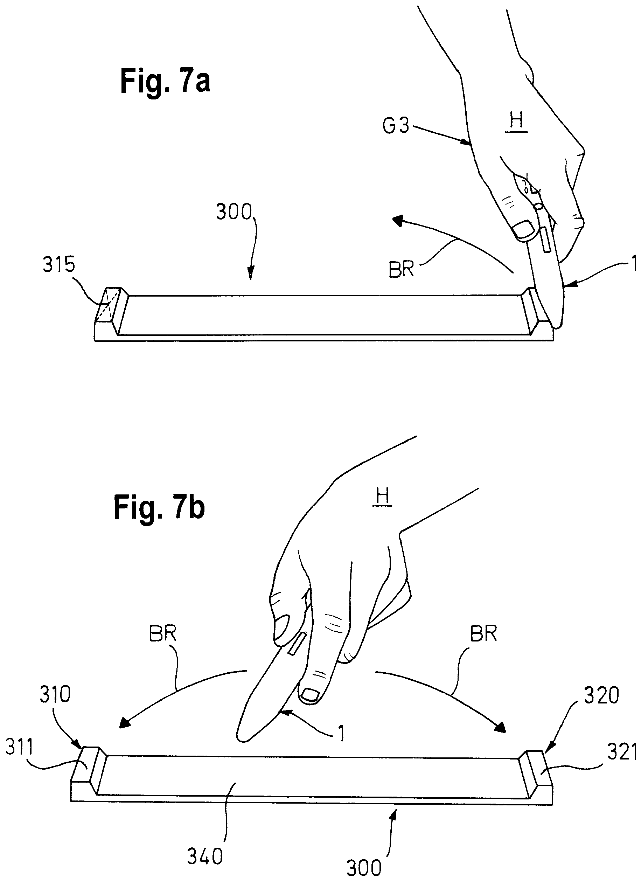

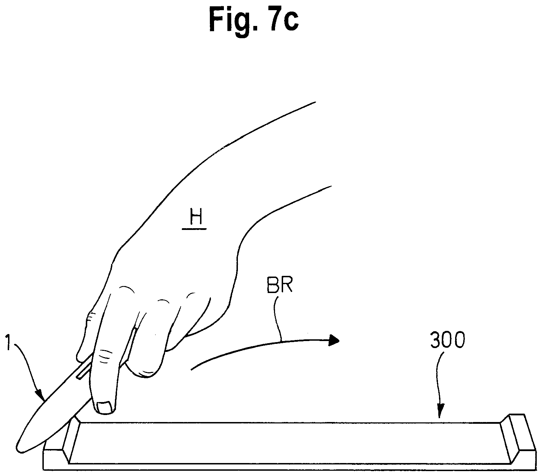

In Fig. 7a bis 7c wird ein beispielhafter Bewegungsablauf eines Targeting-Tests dargestellt. Dabei wird die Vorrichtung 1 in der Hand H eines Patienten in einem dritten Griff G3 gehalten und von dem einen Anschlagelement 310 zu dem anderen, gegenüberliegendem Anschlagelement 320 einer Testeinheit 300 mittels der Hand H des Patienten geführt.

Die Testeinheit 300 weist zwei Anschlagelemente 310, 320 auf, welche mittels eines Verbindungsstegs 340 miteinander verbunden sind. An der Oberfläche der Anschlagelemente 310, 320 befindet sich eine Kontaktfläche 311,312. In der Mitte der Kontaktfläche 311, 321 ist jeweils ein Zielpunkt 315, 325 vorhanden.

Das Anschlagelement 310, 320 kann zusätzlich mit einem Annäherungssensor ausgestattet sein, wobei dieser erkennt, wie nahe die Vorrichtung 1 zum Zeitpunkt des Anschlags der Vorrichtung 1 mit dem Anschlagelement 310, 320 an den Zielpunkt 315, 325 heran geführt wurde.

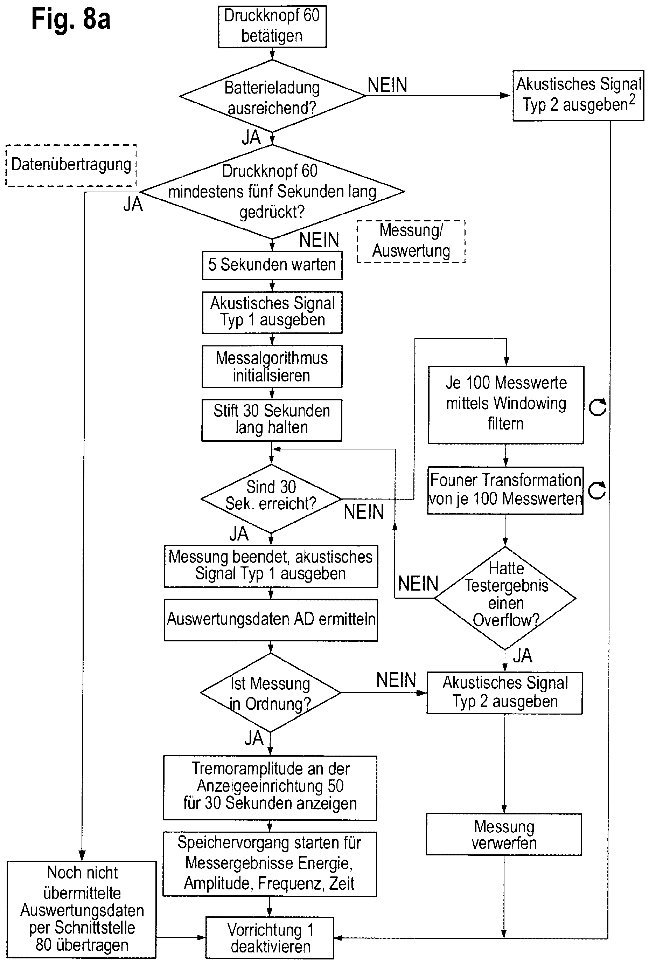

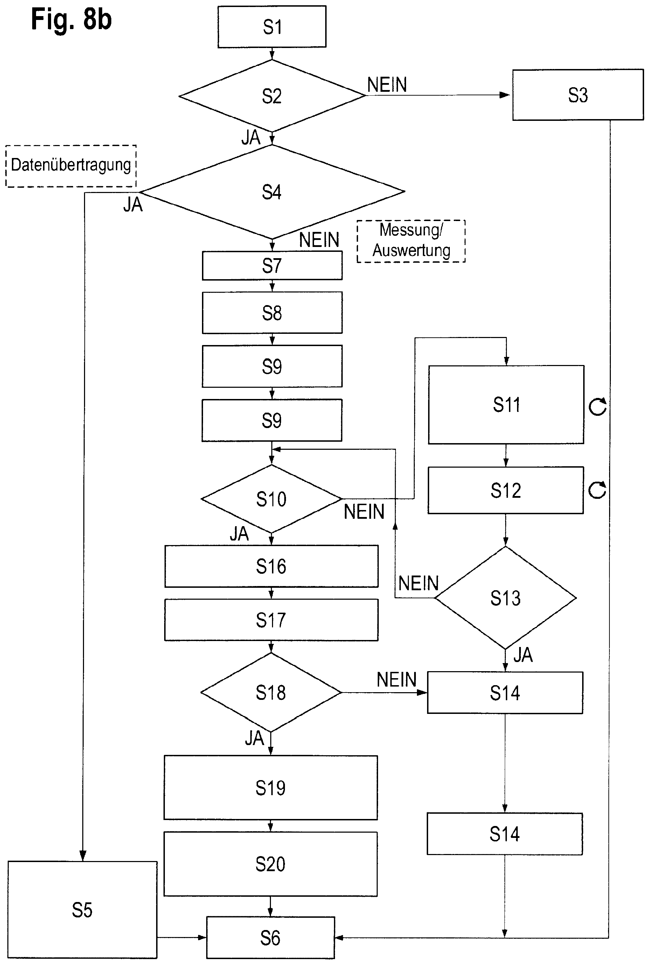

Die Fig. 8a und 8b zeigen jeweils ein Flussdiagramm eines Mess-/Auswertungsvorgangs der Vorrichtung 1. In Schritt S1 wird der Druckknopf 60 der Vorrichtung 1 betätigt. Anschließend wird in einem Schritt S2 geprüft, ob die Ladung der Batterie 70 für einen Mess-/Auswerteprozess ausreicht. Falls die Ladung der Energie nicht ausreicht gibt die Signaleinheit 90 in Schritt S3 ein akustisches Signal von Typ 2 aus, wobei ein akustisches Signal von Typ 2 mehrere schnell hintereinander ab folgende Töne sind. Das Verfahren geht anschließend zu Schritt S6 und die Vorrichtung 1 wird deaktiviert.

Falls die Ladung der Batterie 70 für einen Mess-/Auswerteprozess ausreicht, gibt die Signaleinheit 90 kein akustisches Signal von Typ 2 aus und der Patient kann in Schritt S4 den Druckknopf 60 für mindestens fünf Sekunden, also lange, drücken, um eine Datenübertragung, vorteilhafterweise mittels Bluetooth, einzuleiten. Folglich werden in Schritt S5 in der Speichereinrichtung 40 gespeicherte, noch nicht übermittelte Auswertungsdaten AD über die drahtlose Schnittstelle 80 mit einer externe Datenverarbeitungsvorrichtung ausgetauscht. Das Verfahren springt danach zu Schritt S6 und die Vorrichtung 1 wird deaktiviert.

Wird hingegen in Schritt S4 der Druckknopf 60 nicht lange, sondern nur kurz gedrückt, springt das Verfahren zu Schritt S7. Je nach Ausgestaltung ist es möglich, dass auch in diesem Fall die Auswertungsdaten AD parallel über die drahtlose Schnittstelle 80 an eine externe Datenverarbeitungsvorrichtung übermittelt werden. In Schritt S7 wird die über den Schieberegler eingestellte Messprozedur erfasst und es wird weitere fünf Sekunden lang gewartet, damit der Patient Vorbereitungszeit hat, um die Vorrichtung 1 anleitungsgemäß in der Messposition zu halten. Anschließend wird mit Schritt S8 fortgefahren.

In Schritt S8 gibt die Signaleinheit 90 ein akustisches Signal von Typ 1 aus, das anzeigt, dass die Messung beginnt. Das akustische Signal von Typ 1 zeichnet sich durch die Wiedergabe eines einzelnen Tons aus. Anschließend wird mit Schritt S9 fortgefahren.

In Schritt S9 erfasst der Beschleunigungssensor 20 Messdaten MD über den Tremor. Das Erfassen der Messdaten MD dauert eine Messdauer T1_{T}, T1_{B} oder T1_{D} von ca. 10, 30 und/oder 60 Sekunden. Innerhalb der Messdauer T1 beginnt die Auswertungseinrichtung 30 bereits erfasste Messdaten MD auszuwerten. Das Verfahren springt zu Schritt S10.

In Schritt S10 wird überprüft, ob die Messdauer T1 bereits abgelaufen ist. Ist Die Messdauer T1 noch nicht abgelaufen springt das Verfahren zu Schritt S11.

In Schritt S11 werden je 100 Messdaten MD durch die Auswertungseinrichtung 30 mittels Windowing gefiltert, das heißt gefenstert. Anschließend geht das Verfahren zu Schritt S12 über.

In Schritt S12 werden die je 100 zuvor in Schritt S11 gefensterten Messdaten MD durch die Auswertungseinrichtung 30 Fourier transformiert. Anschließend springt das Verfahren zu Schritt S13.

Für jedes Teilergebnis wird in Schritt S13 überprüft, ob ein sogenannter Overflow vorliegt, also ob ein Teilergebnis derart unrealistisch erscheint, dass es sich um einen Messfehler handelt, der wahrscheinlich durch eine Fehlbenutzung der Vorrichtung 1 hervorgerufen wurde. Solch ein Messfehler kann sowohl durch ein unrealistisch zu hohes, als auch eine unrealistisch zu niedriges Teilergebnis auftreten. Tritt solch eine Overflow auf, springt das Verfahren zu Schritt S14.

In Schritt S14 gibt die Signaleinheit 90 ein akustisches Signal von Typ 2 aus. Es wird zudem festgestellt, dass ein Messfehler aufgetreten ist, die Messdaten MD werden verworfen, und das Verfahren springt zu Schritt S6, in dem die Vorrichtung 1 deaktiviert wird.

Tritt in Schritt S13 kein Overflow auf und ist die Messdauer T1 in Schritt S10 abgelaufen, dann springt das Verfahren zu Schritt S16.

In Schritt S16 gibt die Signaleinheit 90 ein akustisches Signal vom Typ 1 aus und das Erfassen von Messdaten MD durch den Beschleunigungssensor 20 wird beendet. Anschließend springt das Verfahren zu Schritt S 17.

In Schritt S17 ermittelt die Auswertungseinrichtung 30 für den Auswertungszeitraum T2 verschiedene Auswertungsdaten AD, wie die Amplitude des Tremors, die Frequenz des Tremors und/oder der Energiegehalt beziehungsweise die Energie des Tremors. Anschließend springt das Verfahren zu Schritt S18.

In Schritt S18 wird überprüft, ob die Messung und Auswertung in Ordnung ist. Falls ein Fehler realistisch erscheint, springt das Verfahren zu Schritt S14, ein akustisches Signal von Typ 2 wird ausgegeben, die Messung wird verworfen und die Vorrichtung 1 wird in Schritt S6 deaktiviert. Stellt sich die Messung und Auswertung als in Ordnung heraus, spring das Verfahren zu schritt S19.

In Schritt S19 wird von sämtlichen Auswertungsdaten AD bevorzugt nur ein einziger Parameter des Tremors, etwa die Amplitude, in der Einheit mg, die Frequenz oder die Energie, in der Einheit mJ, mittels der Anzeigeeinrichtung 50 für einen Anzeigezeitraum T3 von vorzugsweise ca. 30 Sekunden ausgegeben. Die weiteren Auswertungsdaten AD werden nicht angezeigt.

Zeitgleich werden in Schritt S20 sämtliche Auswertungsdaten AD in der Speichereinrichtung 40 gespeichert. Das Verfahren springt anschließend zu Schritt S6, und die Vorrichtung 1 wird deaktiviert.

**Bezugszeichenliste**

| | | | |
|---|---|---|---|
| 1 | Vorrichtung | 311 | Kontaktfläche |
| 10 | Gehäuse | 315 | Zielpunkt |
| 11 | vorderer Abschnitt | 320 | Anschlagelement |
| 12 | mittlerer Abschnitt | 321 | Kontaktfläche |
| 13 | hinterer Abschnitt | 325 | Zielpunkt |
| 14 | Führungsschiene | 340 | Verbindungssteg |
| 15 | Führungsschiene | 350 | Erfassungseinheit |
| 16 | Öffnung | 360 | Display |
| 17 | Öffnung | 370 | Leuchtelement |
| 18 | Deckel | 380 | Lautsprecher |
| 19 | Vertiefung | | |
| 20 | Beschleunigungssensor | AP1 | erster Abstützpunkt |
| 25 | Drehratensensor | AP2 | zweiter Abstützpunkt |
| 30 | Auswertungseinrichtung | AP3 | Referenzpunkt |
| 40 | Speichereinrichtung | H | Hand |
| 50 | Anzeigeeinrichtung | F | Finger |
| 60 | Aktivierungseinrichtung | Z | Zeigefinger |
| 70 | Batterie | D | Daumen |
| 80 | drahtlose Schnittstelle | L | Länge |
| 90 | Signaleinheit | d | Durchmesser |
| 100 | Platine | MD | Messdaten |
| 200 | externes Gerät | AD | Auswertungsdaten |
| | | X | Längsrichtung |
| 300 | Testeinheit | S | Schwerpunkt |
| 310 | Anschlagelement | T1 | Messdauer |
| T2 | Auswertungsdauer | BR | Bewegungsrichtung |
| T3 | Anzeigedauer | G1 | erste Haltung |
| 0 | erste Einstellposition | G2 | zweite Haltung |
| T | zweite Einstellposition | G3 | dritte Haltung |
| B | dritte Einstellposition | | |
| D | vierte Einstellposition | | |

## Patentansprüche

1. Vorrichtung zum Erfassen und Auswerten von Tremor und Bradykinese im Hand- oder Armbereich eines Patienten, die bei der Benutzung in nur einer Hand (H) des Patienten gehalten wird und umfasst:
ein sich in einer Längsrichtung (x) erstreckendes Gehäuse (10), das im wesentlichen rohrförmig ausgestaltet ist und einen in der Längsrichtung (x) vorderen Abschnitt (11), einen in der Längsrichtung (x) mittleren Abschnitt (12) und einen in der Längsrichtung (x) hinteren Abschnitt (13) aufweist;
wobei das Gehäuse (10) in dem vorderen Abschnitt (11) wenigstens einen ersten Abstützpunkt (AP1) für das dritte Fingerglied (Phalanx distalis) von wenigstens einem Finger (F) der Hand (H) des Patienten und in dem hinteren Abschnitt (13) wenigstens einen zweiten Abstützpunkt (AP2) in dem Zwischenraum zwischen Daumen (D) und Zeigefinger (Z) der Hand (H) des Patienten bildet;
einen Beschleunigungssensor (20) zum Erfassen von Messdaten (MD) über zumindest den Tremor, der in dem Gehäuse (10) angeordnet ist;
eine Auswertungseinrichtung (30) zum autarken Auswerten der Messdaten (MD) und Bereitstellen von Auswertungsdaten (AD), die in dem Gehäuse (10) angeordnet ist;
eine Speichereinrichtung (40) zum Speichern der Messdaten (MD) oder der Auswertungsdaten (AD), die in dem Gehäuse (10) angeordnet ist;
eine Anzeigeeinrichtung (50) zum Anzeigen der Auswertungsdaten (AD), die an dem Gehäuse (10) angeordnet ist;
eine Aktivierungseinrichtung (60) zum Aktivieren von zumindest dem Beschleunigungssensor (20), der Auswertungseinrichtung (30), der Speichereinrichtung (40) oder der Anzeigeeinrichtung (50), die an dem Gehäuse (10) angeordnet ist, und
eine Batterie (70) zum Versorgen von zumindest dem Beschleunigungssensor (20), der Auswertungseinrichtung (30), der Speichereinrichtung (40), der Anzeigeeinrichtung (50) oder der Aktivierungseinrichtung (60) mit elektrischer Energie;
und weiter
eine Signaleinheit (90), die geeignet ist, zumindest ein akustisch oder haptisch wahrnehmbares Signal zu erzeugen;
einen an dem Gehäuse (10) angeordneten Referenzpunkt (AP3) für das dritte Fingerglied von wenigstens einem Finger (F) der Hand (H) des Patienten, der als Tippmulde ausgebildet ist und ein für den Patienten haptisch wahrnehmbares Merkmal bildet, und
einen durch die Aktivierungseinrichtung (60) aktivierbaren Drehratensensor (25), der in dem vorderen Abschnitt (11) des Gehäuses (10) angeordnet ist;
wobei der Beschleunigungssensor (20) in dem vorderen Abschnitt (11) des Gehäuses (10) in räumlicher Nähe zu dem Drehratensensor (25) angeordnet ist.

2. Vorrichtung nach Anspruch 1, **dadurch gekennzeichnet, dass** die Batterie (70) dazu dient, den Drehratensensor (25) mit elektrischer Energie zu versorgen;
wobei vorzugsweise der Beschleunigungssensor (20) und/oder der Drehratensensor (25) eine Abtastrate von jeweils mindestens 50 Hz, vorzugweise mindestens 100 Hz, am bevorzugtesten mindestens 200 Hz, aufweisen.

3. Vorrichtung nach Anspruch 1 oder 2, **dadurch gekennzeichnet, dass** der Beschleunigungssensor (20) und der Drehratensensor (25) auf einer zentralen Achse, die parallel zur Längsrichtung (x) verläuft, angeordnet sind;
wobei vorzugsweise der Beschleunigungssensor (20) und der Drehratensensor (25) eine räumliche Einheit bilden.

4. Vorrichtung nach einem der Ansprüche 1 bis 3, **dadurch gekennzeichnet, dass** der Referenzpunkt (AP3) mittels einer zusätzlich auf dem Gehäuse (10) angebrachten Vorrichtung dargestellt ist.

5. Vorrichtung nach einem der Ansprüche 1 bis 4, **dadurch gekennzeichnet, dass** die Aktivierungseinrichtung (60) einen Druckknopf und/oder einen Schieberegler aufweist; wobei vorzugsweise die Aktivierungseinrichtung (60) wenigstens eine der folgenden Einstellpositionen (0, T, B, D) hat:
eine erste Einstellposition (0), die den Ausschaltzustand definiert;
eine zweite Einstellposition (T), die einen Modus zur Messung von Tremor definiert;
eine dritte Einstellposition (B), die einen Modus zur Messung von Bradykinese definiert;
eine vierte Einstellposition (D), die einen Modus zur differentialdiagnostischen Messung definiert.

6. Vorrichtung nach einem der Ansprüche 1 bis 5, **dadurch gekennzeichnet, dass** die Auswertungsdaten (AD) eine Amplitude des Tremors oder eine Frequenz des Tremors oder einen Energiegehalt des Tremors umfassen;
wobei vorzugsweise die Auswertungsdaten (AD) ferner zumindest eines der folgenden Parameter umfassen: die Anzahl, die Intensität, die Änderung der Stärke und den Rhythmus von Tappings;
wobei vorzugsweise die Auswertungsdaten (AD) ferner zumindest eines der folgenden Parameter umfassen: die Anzahl, die Intensität, die Änderung der Stärke und den Rhythmus von Änderungen der Rotationsrichtung;
wobei vorzugsweise die Auswertungsdaten (AD) ferner zumindest eines der folgenden Parameter umfassen: die Anzahl, die Intensität, die Änderung der Stärke und den Rhythmus von Zieltreffern;
wobei vorzugsweise eine drahtlose Schnittstelle (80) vorgesehen ist, um Auswertungsdaten (AD), Messdaten (MD) und/oder Einstellungen der Aktivierungseinrichtung (60) mit einem externen Gerät (200) auszutauschen.

7. Vorrichtung nach einem der Ansprüche 1 bis 6, **dadurch gekennzeichnet, dass** das Gehäuse (10) eine Länge (L) zwischen 10 cm und 20 cm, insbesondere zwischen 14 cm und 17 cm, in der Längsrichtung (x) hat und vorzugsweise einen Durchmesser (d) zwischen 10 mm und 40 mm, insbesondere zwischen 15 mm und 30 mm, aufweist;
wobei vorzugsweise der vordere Abschnitt (11) zwischen 10% und 60% der Länge (L) aufweist, der mittlere Abschnitt (12) zwischen 10% und 60% der Länge (L) aufweist und der hintere Abschnitt (13) zwischen 10% und 60% der Länge (L) aufweist;
wobei ferner vorzugsweise das Gehäuse (10) zumindest in dem vorderen Abschnitt (11) sich in der Längsrichtung (x) konisch verjüngend ausgestaltet ist.

8. Vorrichtung nach einem der Ansprüche 1 bis 7, **gekennzeichnet durch** wenigstens eine Platine (100), auf welcher der Beschleunigungssensor (20), der Drehratensensor (25), die Auswertungseinrichtung (30), die Speichereinrichtung (40), die Anzeigeeinrichtung (50), die Aktivierungseinrichtung (60), die Batterie (70), die drahtlose Schnittstelle (80) und/oder die Signaleinheit (90) angeordnet sind;
wobei vorzugsweise die Platine (100) formschlüssig oder kraftschlüssig in dem Gehäuse (10) gehalten ist;
wobei vorzugsweise das Gehäuse (10) wenigstens eine Führungsschiene (14, 15) zum Aufnehmen der Platine (100) aufweist;
wobei vorzugsweise der Beschleunigungssensor (20) und der Drehratensensor (25) auf der Platine (100) nebeneinander angeordnet sind, wobei vorzugsweise die Signaleinheit (90) als ein Lautsprecher oder ein Vibrationsmotor ausgestaltet ist.

9. Vorrichtung nach einem der Ansprüche 1 bis 8, **dadurch gekennzeichnet, dass** die Anzeigeeinrichtung (50) in dem mittleren Abschnitt (12) und vorzugsweise die Batterie (70) in dem hinteren Abschnitt (13) angeordnet sind;
wobei ferner vorzugsweise das Gehäuse (10) mit wenigstens einer Öffnung (16, 17) zur Aufnahme der Anzeigeeinrichtung (50), der Aktivierungseinrichtung (60) und/oder des Signaleinheit (90) versehen ist.

10. Vorrichtung nach einem der Ansprüche 1 bis 9, **dadurch gekennzeichnet, dass** das Gehäuse (10) einen abnehmbaren Deckel (19) zum Einlegen der Batterie (70) aufweist;
wobei vorzugsweise die Platine (100) durch den Deckel (19) in dem Gehäuse (10) eingespannt ist.

11. Vorrichtung nach einem der Ansprüche 1 bis 13, **gekennzeichnet durch** einen Schwerpunkt (S), der sich in dem mittleren Abschnitt (12) befindet.

12. Verfahren zum Erfassen und Auswerten von Tremor im Hand- oder Armbereich eines Patienten mit Hilfe von einer Vorrichtung (1) nach einem der Ansprüche 1 bis 11, umfassend die Schritte:
Aktivieren der Vorrichtung (1) mittels der Aktivierungseinrichtung (60) zum Messen von Tremor;
Halten der Vorrichtung (1) derart, dass das dritte Fingerglied (Phalanx distalis) von wenigstens einem Finger (F) der Hand (H) des Patienten an dem wenigstens einen ersten Abstützpunkt (AP1), den das Gehäuse (10) bildet, und der hintere Abschnitt (13) des Gehäuses (10) in dem Zwischenraum zwischen Daumen (D) und Zeigefinger (Z) der Hand (H) des Patienten an dem wenigstens einen zweiten Abstützpunkt (AP2), den das Gehäuse (10) bildet, abgestützt wird und dass das dritte Fingerglied von wenigstens einem Finger (F) der Hand (H) des Patienten an dem Referenzpunkt (AP3) anliegt;
Messen von Messdaten (MD) über den Tremor mittels des Beschleunigungssensors (20) während einer bestimmten Messdauer (T1_{T});
Auswerten der Messdaten (MD) und Bereitstellen von Auswertungsdaten (AD) mittels der Auswertungseinrichtung (30) während einer vorbestimmten Auswertungsdauer (T2_{T});
Speichern der Messdaten (MD) oder der Auswertungsdaten (AD) mittels der Speichereinrichtung (40);
Anzeigen der Auswertungsdaten (AD) mittels der Anzeigeeinrichtung (50) für eine vorbestimmte Anzeigedauer (T3), die wenigstens 20 Sekunden beträgt, und
Deaktivieren der Vorrichtung (1) nach Ablauf der Anzeigedauer (T3);
wobei durch die Signaleinheit (90) zu Beginn der Messdauer (T1_{T}) oder zu Beginn der Auswertungsdauer (T2_{T}) oder zu Beginn der Anzeigedauer (T3) wenigstens ein Signal abgegeben wird.

13. Verfahren nach Anspruch 12, **dadurch gekennzeichnet, dass** die Messdauer (T1_{T}) weniger als 60 Sekunden, insbesondere weniger als 40 Sekunden, bevorzugt ca. 30 Sekunden, beträgt;
wobei vorzugsweise die Auswertungsdauer (T2_{T}) weniger als 10 Sekunden beträgt, und
wobei vorzugsweise die Anzeigedauer (T3) mehr als 45 Sekunden beträgt.

14. Verfahren nach Anspruch 12 oder 13 , **dadurch gekennzeichnet, dass** die Messdaten (MD) durch die Auswertungseinrichtung (40) gefiltert und vorzugsweise von dem Zeitbereich in den Frequenzbereich transformiert werden.

15. Verfahren nach einem der Ansprüche 12 bis 14, **dadurch gekennzeichnet, dass** wenigstens ein weiteres Signal am Ende der Messdauer (T1_{T}) oder am Ende der Auswertungsdauer (T2_{T}) oder am Ende der Anzeigedauer (T3) abgegeben wird;
wobei vorzugsweise das Signal ein akustisch, visuell oder haptisch wahrnehmbares Signal ist.

## Claims

1. Device for detecting and evaluating tremor and bradykinesis in the hand or arm area of a patient, which, in use, is held in only one hand (H) of the patient and comprises:
a housing (10) extending in a longitudinal direction (x), the housing (10) being substantially tubular and having a front section (11) extending in the longitudinal direction (x), a middle section (12) extending in the longitudinal direction (x), and a back section (13) extending in the longitudinal direction (x);
wherein the housing (10) has in the front section (11) at least one first support point (AP1) for the third phalanx (Phalanx distalis) of at least one finger (F) of the patient's hand (H) and, in the back section (13) at least a second support point (AP2) in the space between the thumb (D) and the index finger (Z) of the patient's hand (H);
an acceleration sensor (20) for acquiring measurement data (MD) about at least the tremor, which is arranged in the housing (10);
an evaluation device (30) for autonomous evaluation of the measurement data (MD) and providing evaluation data (AD), which is arranged in the housing (10);
a memory device (40) for storing the measurement data (MD) or the evaluation data (AD), which is arranged in the housing (10);
a display means (50) for displaying the evaluation data (AD), which is arranged on the housing (10);
activating means (60) for activating at least the acceleration sensor (20), the evaluation device (30), the memory device (40) or the display means (50), which is arranged on the housing (10), and
a battery (70) for powering at least the acceleration sensor (20), the evaluation device (30), the memory device (40), the display means (50), or the activation means (60) with electrical energy; and further
a signal unit (90), which is suitable for transmitting at least an acoustically or haptically perceptible signal;
a reference point (AP3) for the third phalanx of at least one finger (F) of the patient's hand (H), arranged on the housing (10), which is formed as a typing depression and forms a haptically perceptible feature for the patient; and
a rotation rate sensor (25) that can be activated by the activating means (60) and which is arranged in the front section (11) of the housing (10);
wherein the acceleration sensor (20) is disposed in the front section (11) of the housing (10) in close proximity to the rotation rate sensor (25).

2. Device according to claim 1, **characterised in that** the battery (70) serves to supply the rotation rate sensor (25) with electrical energy;
wherein preferably the acceleration sensor (20) and/or the rotation rate sensor (25) each have a sampling rate of at least 50 Hz, preferably of at least 100 Hz, most preferably of at least 200 Hz.

3. Device according to claim 1 or 2, **characterised in that** the acceleration sensor (20) and the rotation rate sensor (25) are arranged on a central axis which is parallel to the longitudinal direction (x);
wherein preferably the acceleration sensor (20) and the rotation rate sensor (25) form a spatial unit.

4. Device according to any one of claims 1 to 3, **characterised in that** the reference point (AP3) is displayed by means of a device additionally mounted on the housing (10).

5. Device according to any one of claims 1 to 4, **characterised in that** the activating means (60) comprises a push button and/or a slider;
wherein preferably the activating means (60) has at least one of the following setting positions (0, T, B, D):
a first setting position (0) defining the off state;
a second setting position (T) defining a mode for measuring tremor;
a third setting position (B) defining a mode for the measurement of bradykinesis;
a fourth setting position (D) defining a mode for differential diagnostic measurement.

6. Device according to any one of claims 1 to 5, **characterised in that** the evaluation data (AD) comprises an amplitude of the tremor or a frequency of the tremor or an energy content of the tremor;
wherein preferably the evaluation data (AD) further comprises at least one of the following parameters: the number, the intensity, the change in the strength, and the rhythm of tappings;
wherein preferably the evaluation data (AD) further comprises at least one of the following parameters: the number, the intensity, the change in the strength, and the rhythm of changes in the direction of rotation;
wherein preferably the evaluation data (AD) further comprises at least one of the following parameters: the number, the intensity, the change in the strength, and the rhythm of target hits;
wherein preferably a wireless interface (80) is provided to exchange the evaluation data (AD), measurement data (MD) and/or settings of the activating means (60) with an external device (200).

7. Device according to any one of claims 1 to 6, **characterised in that** the housing (10) has a length (L) between 10 cm and 20 cm, in particular between 14 cm and 17 cm, in the longitudinal direction (x) and preferably has a diameter (d) between 10 mm and 40 mm, in particular between 15 mm and 30 mm;
wherein preferably the front section (11) has between 10% and 60% of the length (L), the central section (12) has between 10% and 60% of the length (L) and the back section (13) has between 10% and 60% of the length (L);
wherein further preferably the housing (10) is configured to extend tapered in the longitudinal direction (x) at least in the front section (11).

8. Device according to any one of claims 1 to 7, **characterised by** at least one printed circuit board (100), on which the acceleration sensor (20), the rotation rate sensor (25), the evaluation device (30), the memory device (40), the display means (50), the activating means (60), the battery (70), the wireless interface (80) and/or the signal unit (90) are arranged;
wherein preferably the printed circuit board (100) is held positively or non-positively in the housing (10);
wherein preferably the housing (10) comprises at least one guide rail (14, 15) for receiving the printed circuit board (100);
wherein preferably the acceleration sensor (20) and the rotation rate sensor (25) are arranged side by side on the circuit board (100);
wherein preferably the signal unit (90) is configured as a speaker or a vibration motor.

9. Device according to any one of claims 1 to 8, **characterised in that** the display means (50) is arranged in the middle section (12), and preferably the battery (70) is arranged in the back section (13);
wherein further preferably the housing (10) is provided with at least one aperture (16, 17) for receiving the display means (50), the activating means (60) and/or the signal unit (90).

10. Device according to any one of claims 1 to 9, **characterised by**, that the housing (10) comprises a removable cover (19) for inserting the battery (70);
wherein preferably the circuit board (100) is clamped by the cover (19) in the housing (10).

11. Device according to any one of claims 1 to 10, **characterised by** a centre of gravity (S) located in the middle section (12).

12. Method for detecting and evaluating tremor in the hand or arm region of a patient with the aid of a device (1) according to any one of claims 1 to 11, comprising the steps:
activating the device (1) by means of the activating means (60) to measuring tremor;
holding the device (1) in such a way that the third phalanx (phalanx distalis) of at least one finger (F) of the patient's hand (H) is supported on the at least one first support point (AP1) formed by the housing (10), and the back section (13) of the housing (10) is supported in the space between the thumb (D) and index finger (Z) of the hand (H) of the patient at the at least one second support point (AP2) formed by the housing (10), and in that the third phalanx of at least one finger (F) of the patient's hand (H) rests against the reference point (AP3);
measuring measurement data (MD) about the tremor by means of the acceleration sensor (20) during a certain measuring period (T1_{T});
evaluating the measurement data (MD) and providing evaluation data (AD) by means of the evaluation device (30) during a predetermined evaluation period (T2_{T});
storing the measurement data (MD) or the evaluation data (AD) by means of the memory device (40);
displaying the evaluation data (AD) by means of the display means (50) for a predetermined display duration (T3) which is at least 20 seconds; and
deactivating the device (1) after the display duration (T3) has expired;
wherein, by means of the signal unit (90), at the beginning of the measuring duration (T1_{T}) or at the beginning of the evaluation period (T2_{T}) or at the beginning of the display period (T3) at least one signal is emitted.

13. Method according to claim 12, **characterised in that** the measurement time (T1_{T}) is less than 60 seconds, in particular less than 40 seconds, preferably about 30 seconds;
wherein preferably the evaluation duration (T2_{T}) is less than 10 seconds; and
wherein preferably the display duration (T3) is more than 45 seconds.

14. Method according to claim 12 or 13, **characterised in that** the measurement data (MD) is filtered by the evaluation device (30) and is preferably transformed from the time domain to the frequency domain.

15. Method according to any one of claims 12 to 14, **characterized in that** at least one further signal is emitted at the end of the measurement duration (T1_{T}) or at the end of the evaluation duration (T2_{T}) or at the end of the display duration (T3);
wherein preferably the signal is an acoustically, visually or haptically perceptible signal.

## Revendications

1. Dispositif pour détecter et évaluer le tremblement et la bradykinésie dans la région de la main ou du bras d'un patient, lequel dispositif est tenu en service dans une seule main (H) du patient et comprend:
un logement (10) s'étendant dans une direction longitudinale (x), ledit logement (10) étant de forme sensiblement tubulaire et ayant une partie avant (11) dans la direction longitudinale (x), une partie médiane (12) dans la direction longitudinale (x) et une partie arrière (13) dans la direction longitudinale (x);
le boîtier (10) formant, dans la partie avant (11), au moins un premier point d'appui (AP1) pour la troisième phalange distale d'au moins un doigt (F) de la main du patient (H) et, dans la partie arrière (13), au moins un deuxième point d'appui (AP2) dans l'espace entre le pouce (D) et l'index (Z) de la main du patient (H);
un capteur d'accélération (20) pour l'acquisition de données de mesure (MD) concernant au moins la secousse, qui est disposé dans le boîtier (10);
un dispositif d'évaluation (30) pour l'évaluation autonome des données de mesure (MD) et la mise à disposition de données d'évaluation (AD), qui est disposé dans le boîtier (10);
un dispositif de stockage (40) pour le stockage des données de mesure (MD) ou des données d'évaluation (AD), qui est disposé dans le boîtier (10);
un dispositif d'affichage (50) pour l'affichage des données d'évaluation (AD), qui est disposé sur le boîtier (10);
un moyen d'activation (60) pour activer au moins l'un dudit capteur d'accélération (20), dudit moyen d'évaluation (30), dudit dispositif de stockage (40) et dudit moyen d'affichage (50) disposé sur ledit boîtier (10);
et une batterie (70) pour alimenter en énergie électrique au moins l'un dudit capteur d'accélération (20), dudit moyen d'évaluation (30), dudit dispositif de stockage (40), dudit moyen d'affichage (50) et dudit moyen d'activation (60);
et en outre une unité de signal (90) qui est appropriée pour générer au moins un signal perceptible acoustiquement ou haptiquement;
un point de référence (AP3), disposé sur le boîtier (10), pour la troisième phalange d'au moins un doigt (F) de la main (H) du patient, lequel point de référence (AP3) est formé comme un évidement de pointe et forme une caractéristique perceptible haptiquement pour le patient, et un capteur de vitesse de rotation (25) qui peut être activé par le dispositif d'activation (60) et qui est disposé dans la partie avant (11) du boîtier (10);
dans lequel le capteur d'accélération (20) est disposé dans la partie avant (11) du boîtier (10) à proximité spatiale du capteur de vitesse de rotation (25).

2. Dispositif selon la revendication 1, **caractérisé en ce que** la batterie (70) est utilisé pour alimenter en énergie électrique le capteur de vitesse angulaire (25);
dans lequel de préférence le capteur d'accélération (20) et/ou le capteur de vitesse de rotation (25) ont chacun une fréquence d'échantillonnage d'au moins 50 Hz, de préférence d'au moins 100 Hz, de préférence d'au moins 200 Hz.

3. Dispositif selon la revendication 1 ou 2, **caractérisé en ce que** le capteur d'accélération (20) et le capteur de vitesse angulaire (25) sont disposés sur un axe central qui est parallèle à la direction longitudinale (x);
dans lequel de préférence le capteur d'accélération (20) et le capteur de vitesse de rotation (25) forment une unité spatiale.

4. Dispositif selon l'une des revendications 1 à 3, **caractérisé en ce que** le point de référence (AP3) est représenté au moyen d'un dispositif supplémentaire monté sur le boîtier (10).

5. Appareil selon l'une quelconque des revendications 1 à 4, **caractérisé en ce que** ledit moyen d'activation (60) comprend un bouton poussoir et/ou un curseur;
de préférence, ledit moyen d'activation (60) a au moins une des positions de réglage suivantes (0, T, B, D):
une première position de réglage (0) définissant l'état d'arrêt;
une deuxième position de réglage (T) définissant un mode de mesure du tremblement;
une troisième position de réglage (B) définissant un mode de mesure de la bradykinésie;
une quatrième position de réglage (D) définissant un mode de mesure de diagnostic différentiel.

6. Appareil selon l'une quelconque des revendications 1 à 5, **caractérisé en ce que** les données d'évaluation (AD) comprennent une amplitude du tremblement ou une fréquence du tremblement ou un contenu énergétique du tremblement;
de préférence les données d'évaluation (AD) comprennent en outre au moins un des paramètres suivants: le nombre, l'intensité, le changement de force et le rythme des tapotements;
de préférence les données d'évaluation (AD) comprennent en outre au moins un des paramètres suivants: le nombre, l'intensité, le changement de force et le rythme de changements de sens de rotation;
de préférence, lesdites données d'évaluation (AD) comprennent en outre au moins un des paramètres suivants: le nombre, l'intensité, le changement de force et le rythme de coups de cible;
de préférence, une interface sans fil (80) est prévue pour échanger des données d'évaluation (AD), des données de mesure (MD) et/ou des réglages dudit dispositif d'activation (60) avec un dispositif externe (200).

7. Dispositif selon l'une quelconque des revendications 1 à 6, **caractérisé en ce que** le boîtier (10) a une longueur (L) comprise entre 10 cm et 20 cm, en particulier entre 14 cm et 17 cm, dans la direction longitudinale (x) et a de préférence un diamètre (d) compris entre 10 mm et 40 mm, en particulier entre 15 mm et 30 mm;
dans lequel de préférence la section avant (11) a entre 10 % et 60 % de la longueur (L), la section médiane (12) a entre 10 % et 60 % de la longueur (L) et la section arrière (13) a entre 10 % et 60 % de la longueur (L);
dans lequel en outre de préférence le boîtier (10) est conique dans la direction longitudinale (x) au moins dans la section avant (11).

8. Dispositif selon l'une des revendications 1 à 7, **caractérisé par** au moins une carte de circuit imprimé (100), sur laquelle le capteur d'accélération (20), le capteur de vitesse de rotation (25), le dispositif d'évaluation (30), le dispositif de stockage (40), le dispositif d'affichage (50), le dispositif d'activation (60), la batterie (70), l'interface sans fil (80) et/ou l'unité de signalisation (90) sont disposés;
de préférence, la carte de circuit imprimé (100) est maintenue positivement ou négativement dans le boîtier (10);
le boîtier (10) présentant de préférence au moins un rail de guidage (14, 15) pour recevoir la carte à circuits imprimés (100);
le capteur d'accélération (20) et le capteur de vitesse de rotation (25) étant de préférence disposés l'un à côté de l'autre sur la carte à circuits imprimés (100) ;
l'unité de signalisation (90) étant de préférence conçue comme un haut-parleur ou un moteur vibrant.

9. Dispositif selon l'une des revendications 1 à 8, **caractérisé en ce que** le dispositif d'affichage (50) est disposé dans la partie centrale (12) et de préférence la batterie (70) est disposée dans la partie arrière (13);
dans lequel en outre de préférence le boîtier (10) est pourvu d'au moins une ouverture (16, 17) pour recevoir le dispositif d'affichage (50), le dispositif d'activation (60) et/ou l'unité de signalisation (90).

10. Appareil selon l'une des revendications 1 à 9, **caractérisé en ce que** le boîtier (10) comprend un couvercle amovible (19) pour l'insertion de la batterie (70);
de préférence la carte de circuit imprimé (100) étant serrée dans le boîtier (10) par le couvercle (19).

11. Dispositif selon l'une des revendications 1 à 10, **caractérisé par** un centre de gravité (S) situé dans la partie médiane (12).

12. Procédé de détection et d'évaluation du tremblement dans la région de la main ou du bras d'un patient au moyen d'un dispositif (1) selon l'une quelconque des revendications 1 à 11, comprenant les étapes suivantes:
activer le dispositif (1) à l'aide du moyen d'activation (60) de la mesure du tremblement;
tenir le dispositif (1) de telle sorte que la troisième phalange (phalange distale) d'au moins un doigt (F) de la main (H) du patient se trouve au niveau du au moins un premier point d'appui (AP1) formé par le boîtier (10) et la partie arrière (13) du boîtier (10) est soutenue dans l'espace entre le pouce (D) et l'index (Z) de la main (H) du patient au niveau d'au moins un deuxième point d'appui (AP2) formé par le boîtier (10), et en ce que la troisième phalange d'au moins un doigt (F) de la main (H) du patient est en contact avec le point de référence (AP3);
mesurer les données de mesure (MD) concernant la secousse au moyen du capteur d'accélération (20) pendant une période de mesure prédéterminée (T1_{T});
évaluer les données de mesure (MD) et fournir des données d'évaluation (AD) au moyen du dispositif d'évaluation (30) pendant une période d'évaluation prédéterminée (T2_{T});
la mémorisation des données de mesure (MD) ou des données d'évaluation (AD) au moyen du dispositif de stockage (40);
l'affichage des données d'évaluation (AD) au moyen du dispositif d'affichage (50) pendant une durée d'affichage (T3) prédéterminée, qui est d'au moins 20 secondes; et
la désactivation du dispositif (1) après l'écoulement de la durée d'affichage (T3);
dans lequel au moins un signal est émis par l'unité de signal (90) au début de la durée de mesure (T1_{T}) ou au début de la durée d'évaluation (T2_{T}) ou au début de la durée d'affichage (T3).

13. Procédé selon la revendication 12, **caractérisé en ce que** la durée de mesure (T1_{T}) est inférieure à 60 secondes, en particulier inférieure à 40 secondes, de préférence environ 30 secondes;
dans lequel de préférence la durée d'évaluation (T2_{T}) est inférieure à 10 secondes, et dans lequel de préférence la durée d'affichage (T3) est supérieure à 45 secondes.

14. Procédé selon la revendication 12 ou 13, **caractérisée en ce que** les données de mesure (MD) sont filtrées par le dispositif d'évaluation (30) et de préférence transformées du domaine temporel au domaine fréquentiel.

15. Procédé selon l'une quelconque des revendications 12 à 14, **caractérisé en ce qu'**au moins un autre signal est émis à la fin de la durée de mesure (T1_{T}) ou à la fin de la durée d'évaluation (T2_{T}) ou à la fin de la durée d'affichage (T3);
le signal étant de préférence un signal perceptible acoustiquement, visuellement ou haptiquement.
